# FASCICULE DE BREVET EUROPEEN

(11) **EP 3 901 129 B1**
(45) Date de publication et mention de la délivrance du brevet: **14.09.2022**
(21) Numéro de dépôt: 21171847.3
(22) Date de dépôt: 14.03.2016
(51) Int. Cl.: C07C 45/69, C07C 47/222, C07C 49/597, C07C 45/61, C07C 51/38, C07C 62/02, C07C 67/347, C07C 69/675, C07C 69/732, C07C 69/734, C07C 69/757, C07C 45/54, A61P 17/00

(54) **PROCEDE DE SYNTHESE DE NOUVEAUX COMPOSES DERIVES D'ACIDE 3-HYDROXY-CYCLOPENTYL ACETIQUE**
VERFAHREN ZUR SYNTHESE VON NEUEN VERBINDUNGEN, DIE VON 3-HYDROXY-CYCLOPENTYL-ESSIGSÄURE ABGELEITET SIND
METHOD FOR SYNTHESISING NOVEL 3-HYDROXY-CYCLOPENTYL ACETIC ACID DERIVATIVE COMPOUNDS

(30) Priorité: 13.03.2015 FR 1500493
(43) Date de publication de la demande: 27.10.2021
(62) Demande divisionnaire de: 16711192.1
(73) Titulaire: L'OREAL, 75008 Paris (FR)
(72) Inventeur: DALKO, Maria, 78000 Versailles (FR); MARAT, Xavier, 75010 Paris (FR); HITCE, Julien, 93601 Aulnay-sous-Bois (FR); LI, Chao-Jun, Quebec, J4Y 3J6 (CA)
(74) Mandataire: Nony

(56) Documents cités:
- EP-A1- 0 775 102
- JP-A- S6 226 248
- MIDDE SREEKANTH ET AL: "Ti(III)-mediated opening of 2,3-epoxy alcohols to build five-membered carbocycles with multiple chiral centres", TETRAHEDRON LETTERS, PERGAMON, GB, vol. 52, no. 14, 1 février 2011 (2011-02-01), pages 1709-1712, XP028185593, ISSN: 0040-4039, DOI: 10.1016/J.TETLET.2011.02.001 [extrait le 2011-02-04]
- KITAHARA T ET AL: "Synthesis of Methyl Jasmonate and Methyl Cucurbate", AGRICULTURAL AND BIOLOGICAL CHEMISTRY, AGRICULTURAL CHEMICAL SOCIETY OF JAPAN, JP, vol. 51, no. 4, 1 avril 1987 (1987-04-01), pages 1129-1133, XP002053538, ISSN: 0002-1369
- P. DUCOS ET AL: "Coupures thermiques du type retro-Diels et Alder-II", TETRAHEDRON, vol. 29, no. 20, 1 janvier 1973 (1973-01-01), pages 3233-3236, XP055239023, AMSTERDAM, NL ISSN: 0040-4020, DOI: 10.1016/S0040-4020(01)93471-4
- KANG HYUN PARK ET AL: "A Pauson-Khand-Type Reaction between Alkynes and Olefinic Aldehydes Catalyzed by Rhodium/Cobalt Heterobimetallic Nanoparticles:? An Olefinic Aldehyde as an Olefin and CO Source", ORGANIC LETTERS, vol. 6, no. 7, 1 avril 2004 (2004-04-01), pages 1183-1186, XP055271076, US ISSN: 1523-7060, DOI: 10.1021/ol049765s

## Description

La présente invention vise un procédé de préparation de composés dérivés d'acide 3-hydroxy-cyclopentyl acétique ainsi que des composés dérivés d'acide 3-hydroxy-cyclopentyl acétique et l'utilisation de composés dérivés d'acide 3-hydroxy-cyclopentyl acétique pour favoriser la desquamation de la peau et/ou stimuler le renouvellement épidermique et/ou lutter contre le vieillissement de la peau. L'invention concerne également des compositions, notamment cosmétiques, pouvant être employées pour favoriser la desquamation de la peau et/ou stimuler le renouvellement épidermique et/ou lutter contre le vieillissement cutané intrinsèque et/ou extrinsèque.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont se différencier et migrer vers la surface de l'épiderme en constituant les différentes assises de celui-ci, jusqu'à former à la surface de l'épiderme les cornéocytes qui sont des cellules mortes qui s'éliminent par desquamation. Cette perte en surface est compensée par la migration de cellules de l'assise basale vers la surface de l'épiderme. Il s'agit du renouvellement perpétuel de la peau. Une élimination forcée de la couche cornée accélère le renouvellement et permet de lutter contre le vieillissement.

Dans le même temps ces cellules poursuivent leur différenciation dont le dernier stade est le cornéocyte. Il s'agit en fait de cellules mortes qui constituent la dernière couche de l'épiderme, c'est à dire la couche la plus externe encore appelée stratum corneum.

Le vieillissement cutané résultant de facteurs intrinsèques ou extrinsèques se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté ou par l'apparition de télangiectasies.

Certains de ces signes du vieillissement sont plus particulièrement liés au vieillissement intrinsèque ou physiologique, c'est-à-dire au vieillissement "normal" lié à l'âge ou chronobiologique, alors que d'autres sont plus spécifiques du vieillissement extrinsèque, c'est-à-dire du vieillissement provoqué d'une manière générale par l'environnement; il s'agit plus particulièrement du photovieillissement dû à l'exposition au soleil, à la lumière ou à tout autre rayonnement.

L'invention s'intéresse au vieillissement intrinsèque ou physiologique ainsi qu'au vieillissement extrinsèque.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et tannée, et des changements histopathologiques tels qu'une excessive accumulation de matière élastique dans le derme supérieur et une dégénérescence des fibres de collagène.

On connaît dans l'art antérieur divers agents destinés à lutter contre le vieillissement cutané.

Ainsi, le brevet US-A-4603146 décrit l'emploi d'acide rétinoïque et de ses dérivés dans des compositions cosmétiques, en vue de lutter contre le vieillissement cutané.

Par ailleurs, de nombreux brevets et publications (voir par exemple la demande EP-A-413528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les β-hydroxyacides et plus spécialement l'acide salicylique ainsi que ses dérivés pour leur propriétés desquamantes (voir les documents WO-A-93/10756 et US-A-4 767 750).

Tous ces composés ont une action contre le vieillissement de la peau en favorisant la desquamation, c'est-à-dire l'élimination des cellules mortes situées à la surface de la couche cornée de l'épiderme. Cette propriété "desquamante" est aussi appelée, souvent à tort, propriété kératolytique.

Mais ces composés de l'art antérieur présentent également des effets secondaires, qui consistent en des picotements, des tiraillements, des échauffements et des rougeurs désagréables pour l'utilisateur.

On connait également de la demande EP 1 333 021 des compositions cosmétiques ou pharmaceutiques comprenant des dérivés de l'acide jasmonique ainsi que l'utilisation de ces dérivés pour favoriser la desquamation de la peau et/ou stimuler le renouvellement épidermique et/ou lutter contre le vieillissement de la peau.

Le procédé selon la présente invention permet la synthèse de nouveaux composés dérivés d'acide 3-hydroxy-cyclopentyl acétique il permet la synthèse d'une famille de composés présentant une grande diversité structurale qui ne peut pas être obtenue au moyen des réactifs mis en œuvre dans les procédés de l'art antérieur, notamment par la mise en œuvre du jasmonate de méthyle utilisé dans les synthèses de la demande EP 1 333 02 .

Le document par Sreekanth, TETRAHEDRON LETTERS, PERGAMON, GB, vol. 52, no. 14, 1 février 2011 (2011-02-01), décrit un procédé de préparation d'un composé de formule (I), procédé comprenant un intermédiaire alcyne.

Le document par KitaHara, AGRICULTURAL AND BIOLOGICAL CHEMISTRY, AGRICULTURAL CHEMICAL SOCIETY OF JAPAN, JP, vol. 51, no. 4, 1 avril 1987 (1987-04-01), pages 1129-1133, décrit l'addition de malonate sur une double liaison. Le document par Ducos, TETRAHEDRON, vol. 29, no. 20, 1 janvier 1973 (1973-01-01), pages 3233-3236, décrit une addition de malonate sur une double liaison conjuguée ( addition de Michaël). cependant R2 est introduit par la suite.

En outre le procédé selon la présente invention est un procédé respectueux de l'environnement, respectant les principes de la Chimie Verte parmi lesquels on peut citer un nombre limité d'étapes, une économie d'atomes, l'utilisation de solvants renouvelables et à l'impact limité sur l'environnement, une minimisation des déchets plus particulièrement dans le cas où les étapes a) et b) sont effectuées dans le même réacteur.

En outre les composés synthétisés par le procédé selon l'invention présentent des propriétés satisfaisantes en termes de favorisation de la desquamation de la peau et/ou stimulation du renouvellement épidermique.

En outre les composés selon la présente invention utilisés à titre d'agents antivieillissement présentent une action au moins aussi efficace que celle des composés de l'art antérieur, mais ne présentant pas leurs inconvénients.

Ainsi, l'invention a pour but de proposer un nouveau procédé de synthèse de composés dérivés d'acide 3-hydroxy-cyclopentyl acétique à partir d'acroléine, d'un alcyne et d'un dérivé malonate. Ce procédé de synthèse permet l'obtention d'une large famille de composés parmi lesquels certains n'ont jamais été décrits.

L'invention a également pour but de pallier les inconvénients des composés de l'art antérieur et de proposer de nouveaux composés susceptibles de favoriser la desquamation de la peau et/ou de stimuler le renouvellement épidermique, dont l'utilisation n'entraînerait pas de picotements, de tiraillements, d'échauffements ou de rougeurs désagréables pour l'utilisateur.

Ainsi selon un premier aspect, la présente invention vise un procédé de préparation d'un composé de formule (I) : 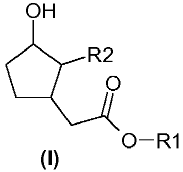 dans laquelle R1 représente un atome d'hydrogène, un radical phényle ou un radical hydrocarboné, linéaire ou ramifié, saturé ou insaturé ayant 1 à 8 atomes de carbone,

R2 désigne un radical choisi parmi :
- un radical hydrocarboné saturé, linéaire, ramifié ou cyclique, ayant 1 à 12 atomes de carbone, éventuellement substitué par 1 à 3 groupements, identiques ou différents, choisis parmi -OR31, -NR31R41, -COOR31, -OCOR31 et les halogènes, avec R31 et R41 représentant, indépendamment l'un de l'autre, un atome d'hydrogène, un radical phényle ou un radical alkyle, linéaire ou ramifié, saturé ou insaturé ayant 1 à 4 atomes de carbone ;
- un radical -Ar-R22 avec Ar est un noyau aromatique choisi parmi les groupes phényle ou pyridyle, et R22 représente un substituant du noyau aromatique Ar choisi parmi : l'hydrogène, -OR', -NO₂, les halogènes, -NH₂, -CF₃ et -R', avec R' désignant un atome d'hydrogène, un radical hydrocarboné linéaire, ramifié ou cyclique, saturé ou insaturé, ayant 1 à 6 atomes de carbone, ou un radical phényle ;
- un radical -R23-Ph dans lequel R23 représente un radical divalent hydrocarboné linéaire ou ramifié, saturé ou insaturé ayant 1 à 6 atomes de carbone, éventuellement substitué par 1 à 3 groupements -OH et/ou éventuellement interrompu par 1 à 3 atomes d'oxygène non adjacents, Ph représentant un groupe phényle ;
- un radical -R24-NH-R34 avec R24 représente un radical divalent hydrocarboné linéaire ou ramifié, saturé ou insaturé ayant 1 à 4 atomes de carbone, éventuellement substitué par 1 à 3 groupements -OH et/ou éventuellement interrompu par 1 à 3 atomes d'oxygène ; R34 représente un substituant -COOR' ou un radical hydrocarboné, linéaire ou ramifié, saturé ou insaturé ayant 1 à 4 atomes de carbone éventuellement interrompu par un groupement NH, O, S, avec R' représente un atome d'hydrogène, un radical hydrocarboné saturé linéaire, ramifié ou cyclique, ayant 1 à 6 atomes de carbone, tel qu'un radical tert-butyle, ou un radical phényle
- un radical -CO-O-R25 dans lequel R25 représente un atome d'hydrogène, un radical phényle ou un radical hydrocarboné, linéaire ou ramifié, saturé ou insaturé ayant 1 à 8 atomes de carbone ;
ainsi que leurs isomères optiques, diastéréoisomères et/ou sels correspondants ; procédé dans lequel les étapes suivantes sont mises en œuvre :
a) addition 1,4 sur l'acroléine d'un alcyne de formule R2C=CH avec R2 tel que défini dans la formule (I) ci-dessus;
b) cyclisation du produit obtenu à l'issu de l'étape à par hydroacylation intramoléculaire ;
c) addition 1,4 sur la cyclopenténone issue de l'étape b) du dérivé malonate CH₂(CO₂CH₂Ph)(CO₂R0) pour lequel, lorsque dans la formule (I) R1 est un atome d'hydrogène, alors -R0 est -CH₂Ph et lorsque dans la formule (I) R1n'est pas un atome d'hydrogène alors R0 est R1 ;
d) réduction et décarboxylation du produit obtenu à l'étape c).

Selon un deuxième aspect, la présente invention concerne l'utilisation pour favoriser la desquamation de la peau et/ou stimuler le renouvellement épidermique et/ou lutter contre le vieillissement de la peau d'un composé de formule (I) dans laquelle R1 et R2 sont tels que définis ci-dessus à l'exclusion des composés de formule (I) pour lesquels R2 est un radical pentyle et R1 est un atome d'hydrogène ou un groupe méthyle.

L'invention sera mieux comprise à la lecture de la description détaillée et des exemples qui suivent.

### Description détaillée

Par « radical hydrocarboné ayant 1 à 12 atomes de carbone », on entend un radical hydrocarboné ayant 1, 2, 3, 4, 5, 6, 7, 8, 9, 10, 11ou 12 atomes de carbone.

Par « un atome d'halogène », on entend un atome choisi parmi F, Cl, Br, I.

La présente invention concerne un nouveau procédé de synthèse de composés dérivés d'acide 3-hydroxy-cyclopentyl acétique à partir d'acroléine, d'un alcyne et d'un dérivé malonate, procédé dans lequel les étapes suivantes sont mises en œuvre :
a) addition 1,4 sur l'acroléine d'un alcyne de formule R2C=CH avec R2 tel que défini dans la formule (I) ci-dessus;
b) cyclisation du produit obtenu à l'issu de l'étape a) par hydroacylation intramoléculaire;
c) addition 1,4 sur la cyclopenténone issue de l'étape b) du dérivé malonate CH₂(CO₂CH₂Ph)(CO₂R0) pour lequel, lorsque dans la formule (I) R1 est un atome d'hydrogène , alors -R0 est -CH₂Ph et lorsque dans la formule (I) R1 n'est pas un atome d'hydrogène alors R0 est R1 ;
d) réduction et décarboxylation du produit obtenu à l'étape c).

Le procédé selon l'invention est particulièrement respectueux de l'environnement, il respecte les principes de la Chimie Verte : nombre limité d'étapes, économie d'atomes afin de minimiser les ressources consommées, mise en œuvre de certains réactifs en quantité catalytique plutôt que stœchiométrique, utilisation de solvants renouvelables et à l'impact limité sur l'environnement, minimisation des déchets dans le cas notamment où les étapes a) et b) sont enchainées dans le même réacteur.

En outre, les substrats de départ, en particulier l'acroléine, sont facilement accessibles.

Ce procédé se caractérise par le fait que la voie de synthèse globale est très directe et qu'il met en œuvre l'acroléine comme produit de départ pour l'addition 1,4 d'un alcyne or l'acroléine est un substrat "difficile" à utiliser car peu stable, très réactif : il tend à polymériser.

Par ailleurs, les conditions opératoires et notamment le système catalytique Pd(OAc)₂/PMe₃ permet justement de favoriser l'addition 1,4 par rapport à la décomposition de l'acroléine.

Ce procédé est résumé dans le schéma 1 ci-dessous :

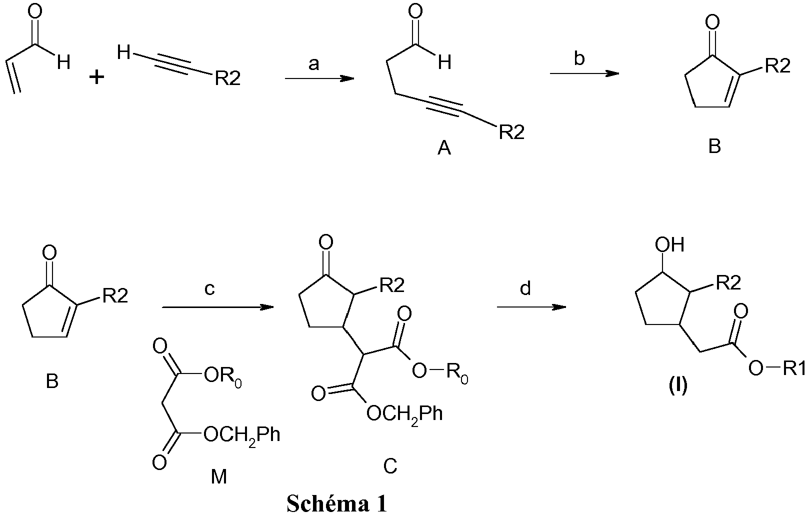

### Etape a)

A l'étape a) est effectuée une addition 1,4 de l'alcyne vrai de formule R2C=CH sur l'acroléine. L'alcyne et l'acroléine sont chauffés à une température comprise entre 30 °C et 140 °C, de préférence entre 100 et 120 °C, dans l'eau ou dans un solvant organique ou dans un mélange d'au moins deux solvants miscibles ou non, en présence d'un catalyseur. De préférence, le solvant est l'eau, l'acétone, un mélange toluène/eau ou un mélange toluène/acétone

Plus particulièrement l'acroléine et l'alcyne R2C=CH sont chauffés à 30-140 °C en présence d'un métal de transition, nommé Mt-a, introduit en quantité catalytique allant de 0.001 équivalent (équiv.) à 0,5 équiv., pendant une durée allant de 5 min à 24 h dans un solvant organique qui est de préférence un mélange d'au moins 2 solvants miscibles ou non choisis parmi le toluène, le benzène, l'eau, le méthanol, l'éthanol, l'isopropanol, l'acétone, la méthyléthylcétone, le THF, le Me-THF, l'éther diéthylique, l'éther diisopropylique, le dichlorométhane et l'acétonitrile.

De façon préférée, l'étape a) est effectuée en présence d'un catalyseur qui est un complexe de palladium en particulier choisi parmi Pd(OAc)₂ , Pd(TFA)₂, Pd(OPiv)₂, PdCl₂, Pd₂dba₃ éventuellement associé à une phosphine ou complexe de rhodium éventuellement associé à une phosphine

Le métal de transition Mt-a peut également être un complexe de rhodium associé à une phosphine.

Avantageusement, le complexe de palladium est associé à une phosphine telle que PMe₃, PPh₃, P(n-Bu)₃ ,PCy₃.

De façon particulièrement préférée, le métal de transition introduit en quantité catalytique est un complexe de palladium associé à la triméthylphosphine. De préférence, le catalyseur est Pd(OAc)₂ et la phosphine est PMe₃. Ils sont avantageusement mise en œuvre dans un rapport molaire Pd(OAc)₂ / PMe₃ 1/3.

Selon une variante, le catalyseur est préalablement formé par chauffage à une température allant de 40à 140 °C de Pd(OAc)₂ en une quantité allant de 0.001 à 0.1 équiv. et PMe₃ en une quantité allant de 0.00 là 0.3 équiv. dans le toluène pendant une durée allant de 2 à 60 min.

Selon une variante, la réaction est conduite dans un mélange de 2 solvants choisis parmi le toluène, le benzène, l'eau, le méthanol, l'acétone, la méthyléthylcétone, le THF, le Me-THF, l'éther diéthylique, l'éther diisopropylique, le dichlorométhane et l'acétonitrile, avantageusement dans un mélange de toluène et d'un solvant plus polaire tel que l'eau, le méthanol, l'acétone, l'acétonitrile.

De façon particulièrement préférée, la réaction est conduite dans un mélange de toluène et d'eau ou un mélange de toluène et d'acétone.

En fin de réaction, le mélange réactionnel est dilué dans un solvant organique tel que l'éther diéthylique, l'éther diisopropylique, l'acétate d'éthyle ou l'acétate d'isopropyle et les phases sont séparées. La phase aqueuse est extraite, par exemple 3 fois avec le même solvant organique et les phases organiques sont rassemblées, séchées sur MgSO4 et concentrées sous pression réduite.

En fin de réaction, le produit obtenu peut être isolé ou non avant de passer à l'étape b.

Dans une variante préférée de l'invention, l'étape a et l'étape b sont réalisées dans le même réacteur sans isoler l'intermédiaire issu de l'étape a. Selon cette variante, de façon encore plus préférée, l'étape a et l'étape b sont réalisées dans le même système de solvants.

Alternativement, si l'on souhaite isoler le produit de la réaction de l'étape a) (intermédiaire A), le résidu peut être purifié selon une méthode classique telle que la chromatographie colonne sur gel de silice.

### Etape b)

A l'étape b) est effectuée une hydroacylation intramoléculaire conduisant à la cyclisation du produit issu de l'étape a). En fin d'étape b) une cyclopenténone est obtenue.

L'intermédiaire A, produit issu de l'étape a) est chauffé à une température comprise entre 40 °C et 140 °C dans un solvant organique en présence d'un catalyseur.

L'adduit issu de l'étape a est chauffé à 40-140 °C en présence d'un métal de transition, nommé Mt-b, identique ou différent de celui utilisé à l'étape a, et introduit en quantité catalytique, de préférence allant de 0,001 équiv. à 0.5 équiv., de préférence pendant une durée allant de 5 min à 24 h dans au moins un solvant. Avantageusement le solvant est choisi parmi le toluène, le benzène, l'eau, le méthanol, l'éthanol, l'isopropanol, l'acétone, la méthyléthylcétone, le THF, le Me-THF, l'éther diéthylique, l'éther diisopropylique, le dichlorométhane et l'acétonitrile.

De façon préférée, le métal de transition [Mt-b] introduit en quantité catalytique est un complexe de palladium associé à une phosphine, un complexe de rhodium associé à une phosphine, un complexe de ruthénium associé à une phosphine ou un complexe d'iridium associé à une phosphine.

De façon particulièrement préférée, le métal de transition introduit en quantité catalytique est un complexe de rhodium associé à une phosphine choisie parmi les phosphines bidentates, telles que l'éthylènebis(diphenylphosphine) (dppe), le 1,4-bis(diphenylphosphino)propane (dppp), le 1,4-bis(diphenylphosphino)butane (dppb), le 1,4-Bis(diphenylphosphino)ferrocène (dppf), la 1,1'-binaphthalene-2,2'-diyl)bis(diphenylphosphine) (BINAP), le 2,2'-Bis(di-p-tolylphosphino)-1,1'-binaphthyl (tol-BINAP). Les conditions de cette réaction d'hydroacylation intramoléculaire catalysée par le rhodium sont en particulier décrites dans Tanaka et Fu (J. Am. Chem. Soc. 2001, 123, 11492*).*

Selon une réalisation particulière, le complexe de rhodium [Rh(dppe)]2(BF4)2 (0.001 équiv. 0.5 équiv.) et l'intermédiaire A (1 équiv.) sont mis en solution dans un solvant tel que l'eau, le toluène, le dichlorométhane, le THF, le Me-THF, l'éther diéthylique, l'éther diisopropylique, le méthanol, l'éthanol, l'acétone, la méthyléthylcétone, l'acétonitrile, sous atmosphère inerte. Le mélange réactionnel est agité pendant une durée allant de 5 min à 72h à une température comprise entre 18 et 150 °C. Après retour à température ambiante, de l'acétonitrile est ajouté et les solvants sont évaporés sous pression réduite. Le résidu est purifié selon une méthode classique telle que la distillation et chromatographie colonne sur gel de silice. La cyclopenténone de formule B est ainsi obtenue.

### Etape c)

A l'étape c) est effectuée une addition 1,4 du dérivé malonate M sur la cyclopenténone de formule B issue de l'étape b). Le dérivé malonate est traité par une base forte dans un solvant organique, puis il est mis en présence de la cyclopenténone.

Plus particulièrement, le dérivé malonate M est traitée par une base forte pendant une durée allant de 5 min à 24 h dans au moins un solvant organique de préférence choisi parmi le N,N-diméthylformamide, le N,N-diméthylacétamide, le méthanol, l'éthanol, l'isopropanol, le tert-butanol, le THF, le Me-THF, l'éther diéthylique, l'éther diisopropylique, le dichlorométhane, puis mis en présence de la cyclopenténone issue de l'étape b)).

De préférence, la base forte est le diéthanolate de magnésium, le tert-butanolate de potassium, le méthylate de sodium, le diisopropylamidure de lithium.

De façon préférée, le solvant est le méthanol, le N,N-diméthylformamide, le tert-butanol ou le THF.

Selon une mise en œuvre préférée de l'invention, 1 équivalent de malonate CH₂(CO₂CH₂Ph)(CO₂R0) est dissous dans un solvant organique qui est de préférence le N,N-diméthylformamide, le N,N-diméthylacétamide, le THF ou l'éther diéthylique.

Puis le malonate M, en quantité allant de 1 à 10 équivalent, est traité, à une température comprise entre -78 °C et 30 °C, par une quantité allant de 1 à 5 équivalents d'un base forte, qui est de préférence choisie parmi le diéthanolate de magnésium, le tert-butanolate de potassium, le méthylate de sodium ou le diisopropylamidure de lithium.

Le milieu réactionnel est agité pendant une durée allant de 5 à 120 min puis 1 équivalent de la cyclopentéonone B préalablement dissous dans le solvant de réaction est ajouté goutte-à-goutte.

Le mélange réactionnel est agité pendant une durée allant de 5 minutes à 24 h puis refroidi à une température comprise entre-30 et 0 °C. L'excès de base est neutralisé par ajout d'une solution aqueuse saturée en NH₄Cl et l'adduit C est extrait, de 1 à 4 fois, à l'aide d'un solvant organique tel que l'éther diéthylique, l'éther diisopropylique, l'acétate d'éthyle ou l'acétate d'isopropyle. Les phases organiques sont rassemblées, séchées sur Na₂SO₄ et concentrées sous pression réduite. Le résidu est purifié par une méthode classique telle qu'une chromatographie colonne sur gel de silice pour isoler l'intermédiaire C.

### Etape d

A l'étape d) une réduction et une décarboxylation de l'intermédiaire C sont effectuées de façon concomitante. L'intermédiaire C est mis au contact d'un donneur d'hydrures en présence d'un catalyseur d'hydrogénation dans au moins un solvant organique. Le mélange réactionnel est ensuite chauffé à une température comprise entre 50 et 200 °C, de préférence entre 100 et 200 °C.

L'adduit C issu de l'étape c) est mis au contact d'un donneur d'hydrures en présence d'un catalyseur d'hydrogénation, nommé Mt-d, introduit en quantité catalytique allant de0.001 équiv. à 0,5 équiv., pendant une durée allant de 5 min à 24 heures dans au moins un solvant choisi de préférence parmi le toluène, le benzène, l'eau, le méthanol, l'éthanol, l'isopropanol, l'acétone, la méthyléthylcétone, le THF, le Me-THF, l'éther diéthylique, l'éther diisopropylique, le dichlorométhane et l'acétonitrile. Le mélange réactionnel est ensuite chauffé à une température comprise entre 50 et 200 °C pendant une durée allant de 5 min à 24 heures dans un autoclave.

De façon préférée, le donneur d'hydrure est le dihydrogène ou le cyclohexène, le solvant est choisi parmi l'eau, le méthanol, l'éthanol, l'isopropanol, l'acétone, la méthyléthylcétone, le THF, le Me-THF, l'éther diéthylique, l'éther diisopropylique, le dichlorométhane.

De façon préférée, le catalyseur d'hydrogénation est à base d'au moins un métal choisi parmi le palladium, le ruthénium, l'iridium, le rhodium. De façon particulièrement préférée, le catalyseur d'hydrogénation est le palladium sur charbon.

Selon une mise en œuvre préférée de l'invention, l'intermédiaire C, préalablement dissous dans un solvant organique tel que le méthanol, l'éthanol, l'isopropanol, le THF, le Me-THF, l'éther diéthylique, l'éther diisopropylique ou le dichlorométhane, est agité à une température comprise entre 18 et 100 °C, sous atmosphère de dihydrogène en présence d'un catalyseur d'hydrogénation tel que le palladium sur charbon, pendant une durée allant de 5 min à 24 h. L'hydrogène est ensuite éliminé par passage d'un flux d'argon et le mélange est chauffé à une température comprise entre 50 et180 °C. Après retour à température ambiante, le catalyseur d'hydrogénation est filtré sur celite. Le filtrat est concentré sous pression réduite et le résidu est purifié par chromatographie colonne sur gel de silice pour l'isoler le composé (I).

Le procédé selon la présente invention permet, en particulier, l'obtention des composés décrits ci-après de manière générale ainsi que l'obtention des familles préférées de composés nouveaux selon la présente invention et des composés individuels nouveaux décrits ci-après, ainsi que de leurs isomères optiques, diastéréoisomères et sels correspondants.

Le procédé selon la présente invention permet, avantageusement, l'obtention des composés de formule (I) pour lesquels R1 est l'hydrogène ou un groupe éthyle, R2 étant tel que défini ci-dessus.

Le procédé selon la présente invention permet, en particulier, l'obtention des composés de formule (I) pour lesquels R1 représente un atome d'hydrogène ou un radical hydrocarboné saturé, linéaire ou ramifié ayant 1 à 4 atomes de carbone, tel qu'un radical éthyle et R2 est un radical hydrocarboné saturé, linéaire, ramifié ou cyclique ayant 1 à 12 atomes de carbone, éventuellement substitué par 1 ou 2 groupements, identiques ou différents, choisis parmi -OR31, NR31R41, COOR31, halogène avec R31 et R41 représentant, indépendamment l'un de l'autre, un atome d'hydrogène ou un radical hydrocarboné saturé, linéaire ou ramifié ayant 1 à 4 atomes de carbone, ainsi que leurs sels, isomères et solvates.

Le procédé selon la présente invention permet, en particulier, l'obtention des composés suivants présentés dans le tableau 1 ci-dessous.

**Tableau 1**

| N ° | Structure R1=H | N ° | Structure R1 = C2H5 |
|---|---|---|---|
| 1 | 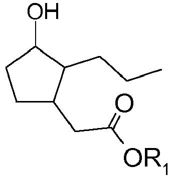 | 1' | 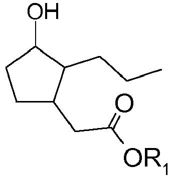 |
| 2 | 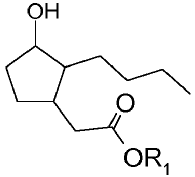 | 2' | 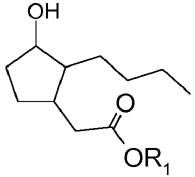 |
| 3 | 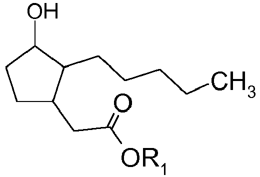 | 3' | 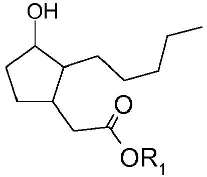 |
| 4 | 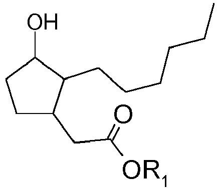 | 4' | 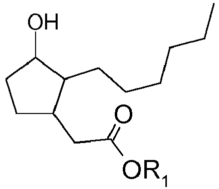 |
| 5 | 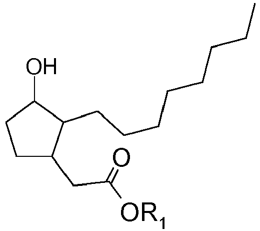 | 5' | 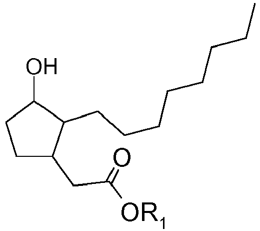 |
| 6 | 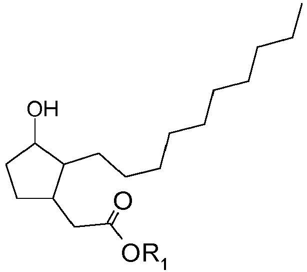 | 6' | 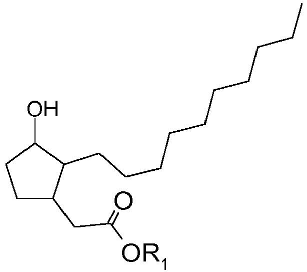 |
| 7 | 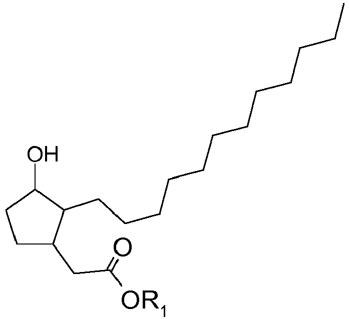 | 7' | 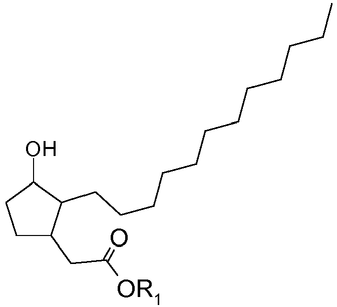 |
| 8 | 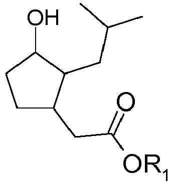 | 8' | 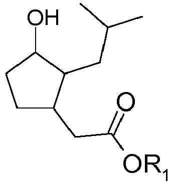 |
| 9 | 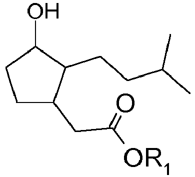 | 9' | 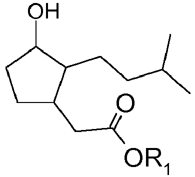 |
| 10 | 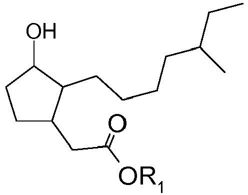 | 10' | 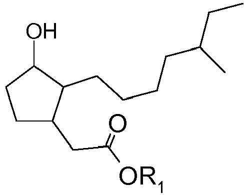 |
| 11 | 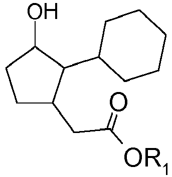 | 11' | 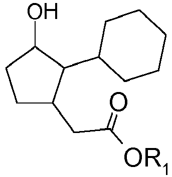 |
| 12 | 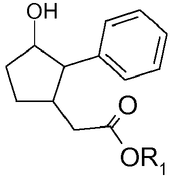 | 12' | 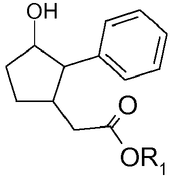 |
| 13 | 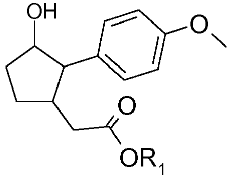 | 13' | 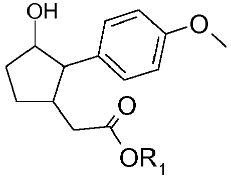 |
| 14 | 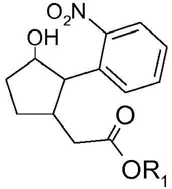 | 14' | 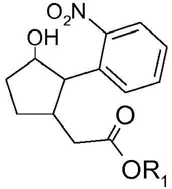 |
| 15 | 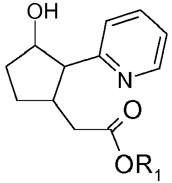 | 15' | 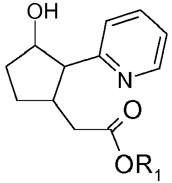 |
| 16 | 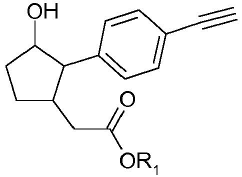 | 16' | 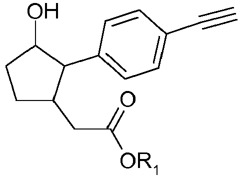 |
| 17 | 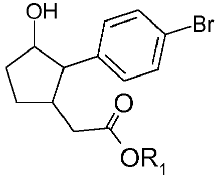 | 17' | 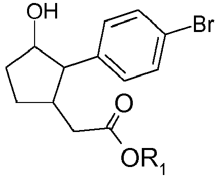 |
| 18 | 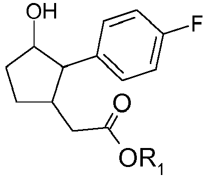 | 18' | 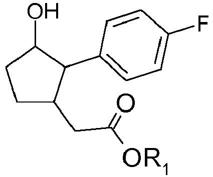 |
| 19 | 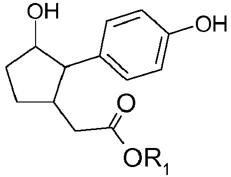 | 19' | 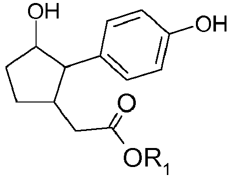 |
| 20 | 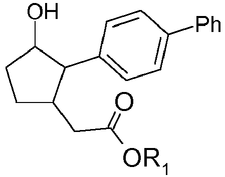 | 20' | 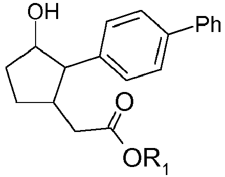 |
| 21 | 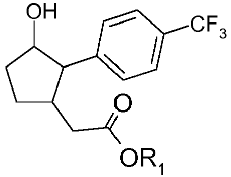 | 21' | 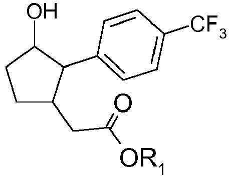 |
| 22 | 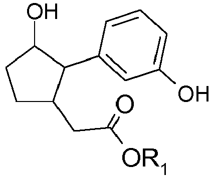 | 22' | 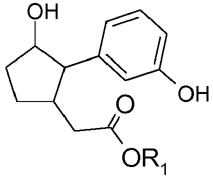 |
| 23 | 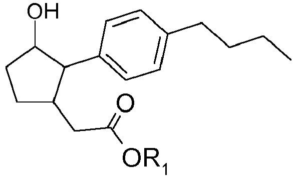 | 23' | 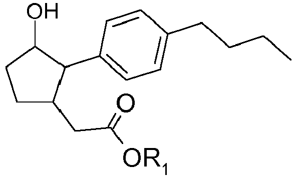 |
| 24 | 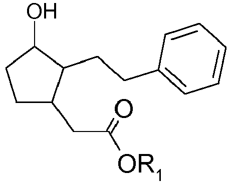 | 24' | 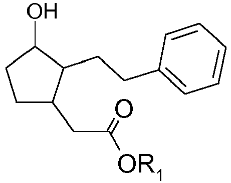 |
| 25 | 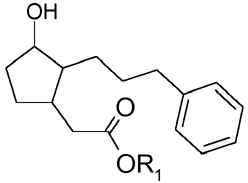 | 25' | 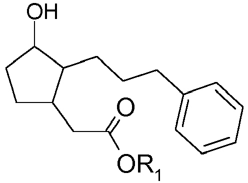 |
| 26 | 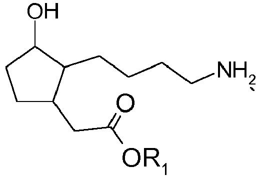 | 26' | 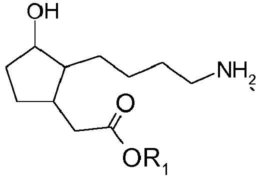 |
| 27 | 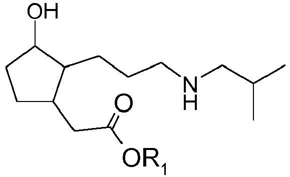 | 27' | 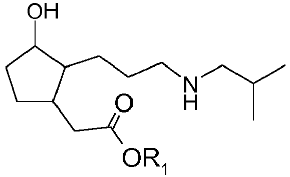 |
| 28 | 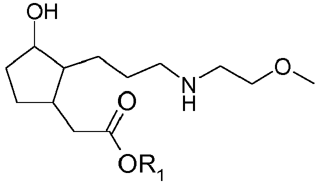 | 28' | 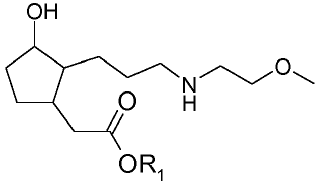 |
| 29 | 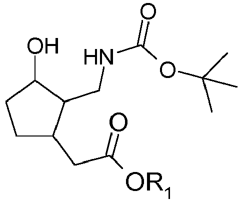 | 29' | 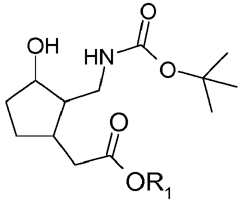 |
| 30 | 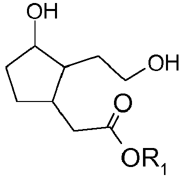 | 30' | 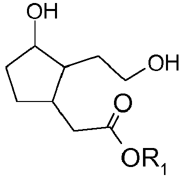 |
| 31 | 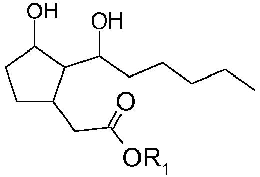 | 31' | 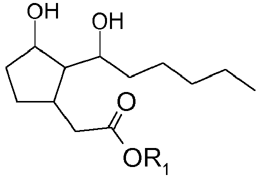 |
| 32 | 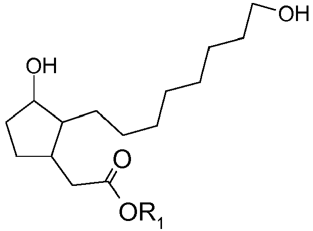 | 32' | 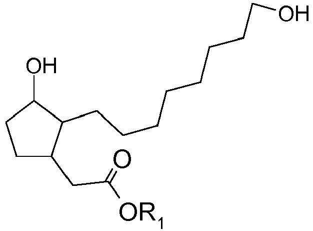 |
| 33 | 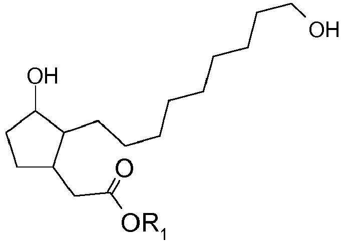 | 33' | 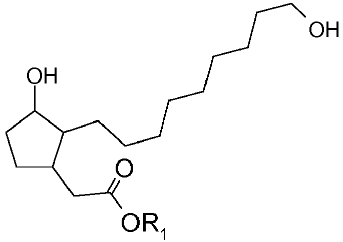 |
| 34 | 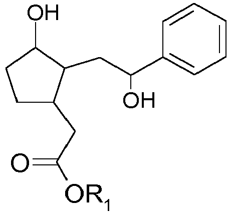 | 34' | 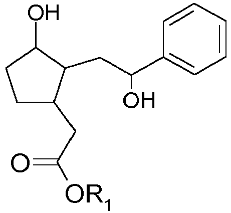 |
| 35 | 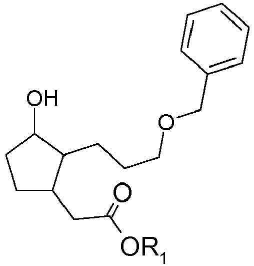 | 35' | 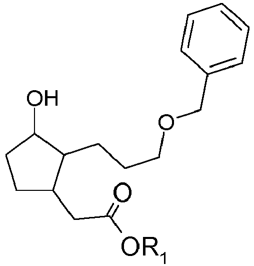 |
| 36 | 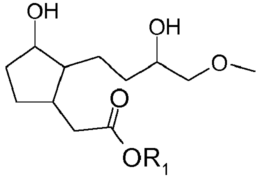 | 36' | 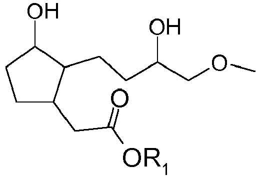 |
| 37 | 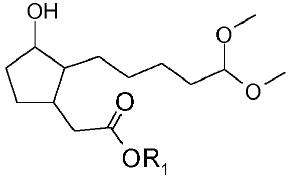 | 37' | 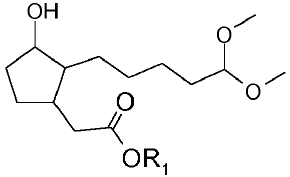 |
| 38 | 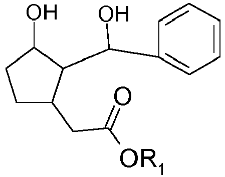 | 38' | 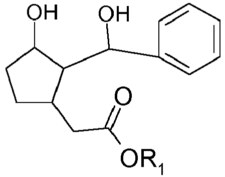 |
| 39 | 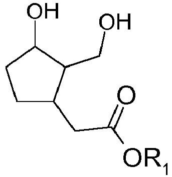 | 39' | 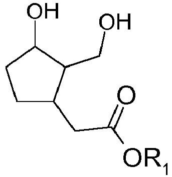 |
| 40 | 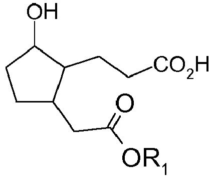 | 40' | 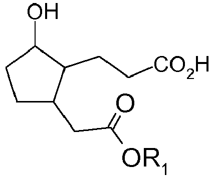 |
| 41 | 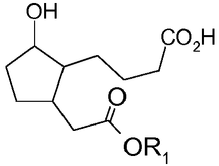 | 41' | 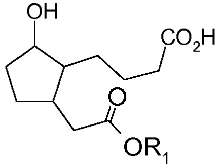 |
| 42 | 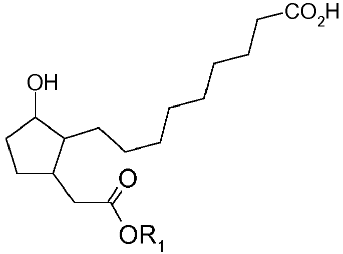 | 42' | 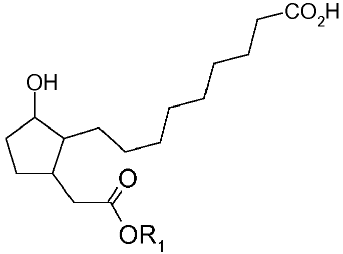 |
| 43 | 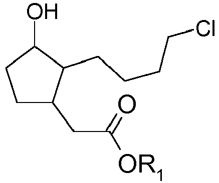 | 43' | 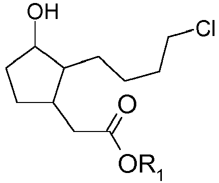 |
| 44 | 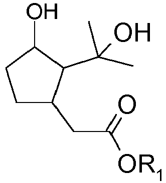 | 44' | 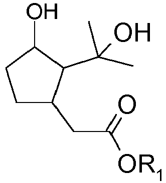 |
| 45 | 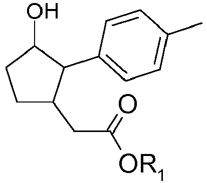 | 45' | 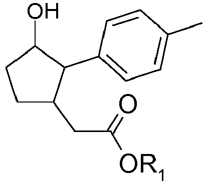 |
| 46 | 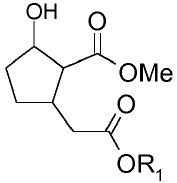 | 46' | 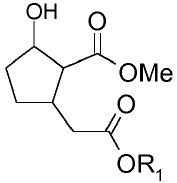 |
| 47 | 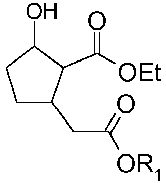 | 47' | 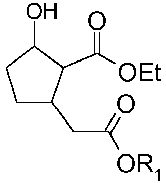 |
| 48 | 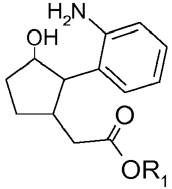 | 48' | 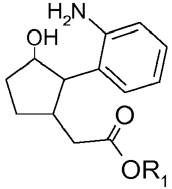 |
| 49 | 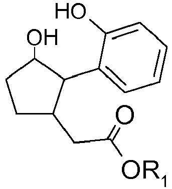 | 49' | 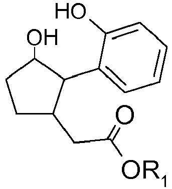 |
| 50 | 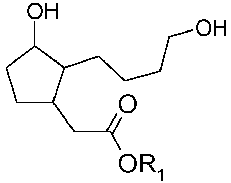 | 50' | 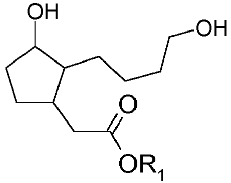 |
| 51 | 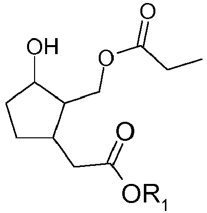 | 51' | 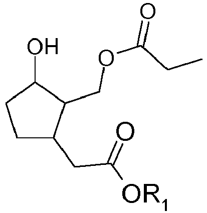 |

ainsi que leurs isomères optiques, diastéréoisomères et/ou sels correspondants.

Le procédé de préparation selon la présente invention permet également l'obtention des composés décrits dans toutes les variantes présentées dans la suite de la description.

Les exemples qui suivant illustrent l'invention sans en limiter la portée.

### EXEMPLES

### I. Préparation des composés

### Exemple 1 : préparation du composé 5

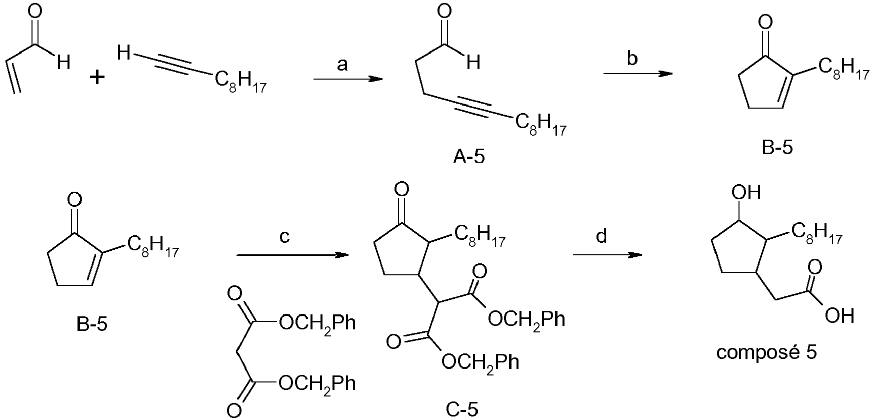

**Etape** a : Une solution 1M de PMe₃ dans le toluène (0,30 équiv., 0,15 mmol, 1 mL) est additionnée à du Pd(OAc)₂ (0,1 équiv., 0,05 mmol, 11,2 mg). Le mélange est chauffé à 110 °C sous argon pendant 10 min, de manière à ce que Pd(OAc)₂ soit complètement dissous. Après retour à température ambiante, le milieu réactionnel est dilué par ajout de 0,5 mL d'eau puis un mélange de 1-décyne (1 équiv., 0.5 mmol) et d'acroléine (5 équiv., 2.5 mmol) est additionné. Le mélange est chauffé à 60 °C pendant 17h (disparition complète du 1-décyne en CCM). Après retour à température ambiante, le mélange réactionnel est dilué dans de l'éther diéthylique et les phases sont séparées. La phase aqueuse est extraite 3 fois avec de l'éther diéthylique puis les phases organiques sont rassemblées, séchées sur MgSO₄ et concentrées sous pression réduite. Le résidu est purifié par chromatographie colonne sur gel de silice (hexane/acétate d'éthyle : 95/5) pour isoler l'intermédiaire A-5 avec un rendement de 62 %.

### Etape b :

L'intermédiaire A-5 est cyclisé par réaction d'hydroacylation intramoléculaire catalysée par le rhodium selon les conditions décrites par Tanaka et Fu (J. Am. Chem. Soc. 2001, 123, 11492) pour ce type de réaction.

Le complexe de rhodium [Rh(dppe)]₂(BF₄)₂ (0.1 équiv.) et l'intermédiaire A-5 (1 équiv.) sont mis en solution dans l'acétone, dans un tube de Schlenk, sous atmosphère inerte. Le tube est scellé et le mélange réactionnel est agité à une température de 90 °C pendant 16h. Après retour à température ambiante, de l'acétonitrile est ajouté et les solvants sont évaporés sous pression réduite. Le résidu est purifié par chromatographie colonne sur gel de silice pour isoler l'intermédiaire B-5.

### Etape c :

5 équivalents du dérivé malonate CH₂(CO₂CH₂Ph)(CO₂CH₂Ph) sont dissous dans le THF.

Puis le dérivé malonate est traité, à une température comprise entre -78 °C et 30 °C, par 5 équivalents d'une base forte : le diéthanolate de magnésium.

Le milieu réactionnel est agité pendant 30min puis 1 équivalent de la cyclopentéonone B-5 préalablement dissous dans le solvant de réaction est ajouté goutte-à-goutte.

Le mélange réactionnel est agité pendant 4h puis refroidi à une température comprise entre -30 et 0 °C. L'excès de base est neutralisé par ajout d'une solution aqueuse saturée en NH₄Cl et l'adduit C-5 est extrait de 3 fois à l'aide d'acétate d'éthyle. Les phases organiques sont rassemblées, séchées sur Na₂SO₄ et concentrées sous pression réduite. Le résidu est purifié par chromatographie colonne sur gel de silice pour isoler l'intermédiaire C-5.

### Etape d :

L'intermédiaire C-5, préalablement dissous dans l'éthanol, est agité à une température comprise entre 18 et 100 °C, sous atmosphère de dihydrogène en présence d'un catalyseur palladium sur charbon, pendant 4 h. L'hydrogène est ensuite éliminé par passage d'un flux d'argon et le mélange est chauffé à 150 °C pendant 4 heures. Après retour à température ambiante, le catalyseur d'hydrogénation est filtré sur celite. Le filtrat est concentré sous pression réduite et le résidu est purifié par chromatographie colonne sur gel de silice pour l'isoler le composé 5.

### Exemple 2 : préparation du composé 9

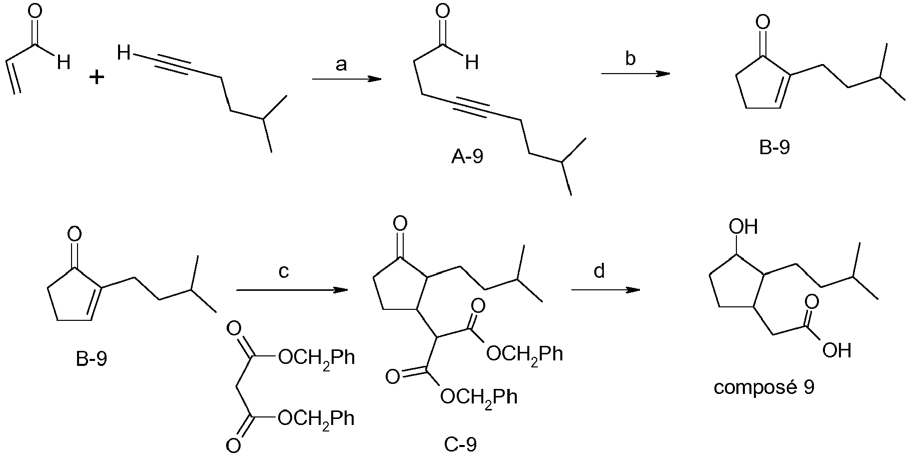

### Etape a :

L'intermédiaire A-9 est obtenu de façon strictement similaire à l'intermédiaire A-5 en substituant le 1-décyne par le 5-méthylhex-1-yne, avec un temps de réaction de 17h. Rendement 51 %.

Le spectre RMN ¹H et le spectre de masse sont en accord avec la structure attendue.

### Etape b :

L'intermédiaire A-9 est cyclisé par réaction d'hydroacylation intramoléculaire catalysée par le rhodium selon les conditions décrites par Tanaka et Fu (J. Am. Chem. Soc. 2001, 123, 11492) pour ce type de réaction.

Le complexe de rhodium [Rh(dppe)]₂(BF₄)₂ (0.1 équiv.) et l'intermédiaire A-9 (1 équiv.) sont mis en solution dans l'acétone, dans un tube de Schlenk, sous atmosphère inerte. Le tube est scellé et le mélange réactionnel est agité à une température de 90 °C pendant 16h. Après retour à température ambiante, de l'acétonitrile est ajouté et les solvants sont évaporés sous pression réduite. Le résidu est purifié par chromatographie colonne sur gel de silice pour isoler l'intermédiaire B-9.

### Etape c :

5 équivalents du dérivé malonate CH₂(CO₂CH₂Ph)(CO₂CH₂Ph) sont dissous dans le THF.

Puis le dérivé malonate est traité, à une température comprise entre -78 °C et 30 °C, par 5 équivalents d'une base forte : le diéthanolate de magnésium.

Le milieu réactionnel est agité pendant 30 min puis 1 équivalent de la cyclopentéonone B-9 préalablement dissous dans le solvant de réaction est ajouté goutte-à-goutte.

Le mélange réactionnel est agité pendant 4h puis refroidi à une température comprise entre -30 et 0 °C. L'excès de base est neutralisé par ajout d'une solution aqueuse saturée en NH₄Cl et l'adduit C-9 est extrait de 3 fois à l'aide d'acétate d'éthyle. Les phases organiques sont rassemblées, séchées sur Na₂SO₄ et concentrées sous pression réduite. Le résidu est purifié par chromatographie colonne sur gel de silice pour isoler l'intermédiaire C-9.

### Etape d :

L'intermédiaire C-9, préalablement dissous dans l'éthanol, est agité à une température comprise entre 18 et 100 °C, sous atmosphère de dihydrogène en présence d'un catalyseur palladium sur charbon, pendant 4 h. L'hydrogène est ensuite éliminé par passage d'un flux d'argon et le mélange est chauffé à 150 °C pendant 4 heures. Après retour à température ambiante, le catalyseur d'hydrogénation est filtré sur celite. Le filtrat est concentré sous pression réduite et le résidu est purifié par chromatographie colonne sur gel de silice pour l'isoler le composé 9.

### Exemple 3 : préparation du composé 12

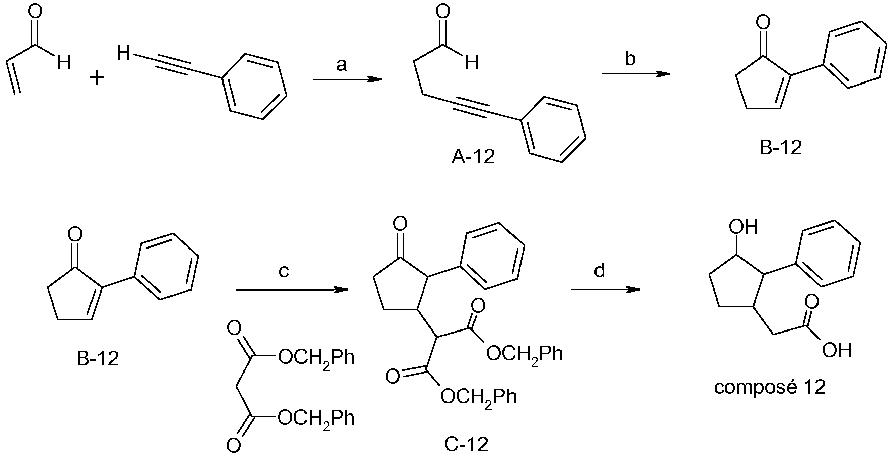

### Etape a :

L'intermédiaire A-12 est obtenu de façon strictement similaire à l'intermédiaire A-5 en substituant le 1-décyne par le phénylacétylène, avec un temps de réaction de 3h. Rendement 70 %.

Le spectre RMN ¹H et le spectre de masse sont en accord avec la structure attendue.

### Etape b :

L'intermédiaire A-12 est cyclisé par réaction d'hydroacylation intramoléculaire catalysée par le rhodium selon les conditions décrites par Tanaka et Fu (J. Am. Chem. Soc. 2001, 123, 11492) pour ce type de réaction.

Le complexe de rhodium [Rh(dppe)]₂(BF₄)₂ (0.1 équiv.) et l'intermédiaire A-12 (1 équiv.) sont mis en solution dans l'acétone, dans un tube de Schlenk, sous atmosphère inerte. Le tube est scellé et le mélange réactionnel est agité à une température de 90 °C pendant 16h. Après retour à température ambiante, de l'acétonitrile est ajouté et les solvants sont évaporés sous pression réduite. Le résidu est purifié par chromatographie colonne sur gel de silice pour isoler l'intermédiaire B-12 (rendement 31 %).

### Etape c :

5 équivalents du dérivé malonate CH₂(CO₂CH₂Ph)(CO₂CH₂Ph) sont dissous dans le THF.

Puis le dérivé malonate est traité, à une température comprise entre -78 °C et 30 °C, par 5 équivalents d'une base forte : le diéthanolate de magnésium.

Le milieu réactionnel est agité pendant 30 min puis 1 équivalent de la cyclopentéonone B-12 préalablement dissous dans le solvant de réaction est ajouté goutte-à-goutte.

Le mélange réactionnel est agité pendant 4h puis refroidi à une température comprise entre -30 et 0 °C. L'excès de base est neutralisé par ajout d'une solution aqueuse saturée en NH₄Cl et l'adduit C-12 est extrait de 3 fois à l'aide d'acétate d'éthyle. Les phases organiques sont rassemblées, séchées sur Na₂SO₄ et concentrées sous pression réduite. Le résidu est purifié par chromatographie colonne sur gel de silice pour isoler l'intermédiaire C-12.

### Etape d :

L'intermédiaire C-12, préalablement dissous dans l'éthanol, est agité à une température comprise entre 18 et 100 °C, sous atmosphère de dihydrogène en présence d'un catalyseur palladium sur charbon, pendant 4 h. L'hydrogène est ensuite éliminé par passage d'un flux d'argon et le mélange est chauffé à 150 °C pendant 4 heures. Après retour à température ambiante, le catalyseur d'hydrogénation est filtré sur celite. Le filtrat est concentré sous pression réduite et le résidu est purifié par chromatographie colonne sur gel de silice pour l'isoler le composé 12.

### Exemple 4 : préparation du composé 13

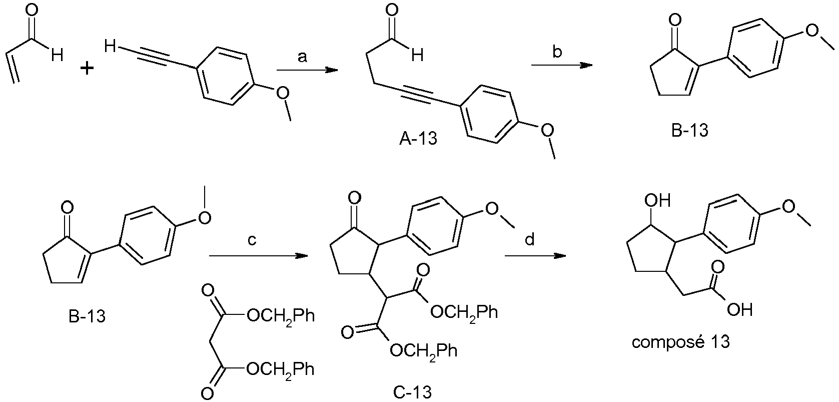

### Etape a :

L'intermédiaire A-13 est obtenu de façon strictement similaire à l'intermédiaire A-5 en substituant le 1-décyne par le (4-méthoxyphényl)acétylène, avec un temps de réaction de 3h. Rendement 73 %.

Le spectre RMN ¹H et le spectre de masse sont en accord avec la structure attendue.

### Etape b :

L'intermédiaire A-13 est cyclisé par réaction d'hydroacylation intramoléculaire catalysée par le rhodium selon les conditions décrites par Tanaka et Fu (J. Am. Chem. Soc. 2001, 123, 11492) pour ce type de réaction.

Le complexe de rhodium [Rh(dppe)]₂(BF₄)₂ (0.1 équiv.) et l'intermédiaire A-13 (1 équiv.) sont mis en solution dans l'acétone, dans un tube de Schlenk, sous atmosphère inerte. Le tube est scellé et le mélange réactionnel est agité à une température de 90 °C pendant 16h. Après retour à température ambiante, de l'acétonitrile est ajouté et les solvants sont évaporés sous pression réduite. Le résidu est purifié par chromatographie colonne sur gel de silice pour isoler l'intermédiaire B-13

### Etape c :

5 équivalents du dérivé malonate CH₂(CO₂CH₂Ph)(CO₂CH₂Ph) sont dissous dans le THF.

Puis le dérivé malonate est traité, à une température comprise entre -78 °C et 30 °C, par 5 équivalents d'une base forte : le diéthanolate de magnésium.

Le milieu réactionnel est agité pendant 30 min puis 1 équivalent de la cyclopentéonone B-13 préalablement dissous dans le solvant de réaction est ajouté goutte-à-goutte.

Le mélange réactionnel est agité pendant 4h puis refroidi à une température comprise entre -30 et 0 °C. L'excès de base est neutralisé par ajout d'une solution aqueuse saturée en NH₄Cl et l'adduit C-13 est extrait de 3 fois à l'aide d'acétate d'éthyle. Les phases organiques sont rassemblées, séchées sur Na₂SO₄ et concentrées sous pression réduite. Le résidu est purifié par chromatographie colonne sur gel de silice pour isoler l'intermédiaire C-13.

### Etape d :

L'intermédiaire C-13, préalablement dissous dans l'éthanol, est agité à une température comprise entre 18 et 100 °C, sous atmosphère de dihydrogène en présence d'un catalyseur palladium sur charbon, pendant 4 h. L'hydrogène est ensuite éliminé par passage d'un flux d'argon et le mélange est chauffé à 150 °C pendant 4 heures. Après retour à température ambiante, le catalyseur d'hydrogénation est filtré sur celite. Le filtrat est concentré sous pression réduite et le résidu est purifié par chromatographie colonne sur gel de silice pour l'isoler le composé 13.

### Exemple 5 : préparation du composé 21

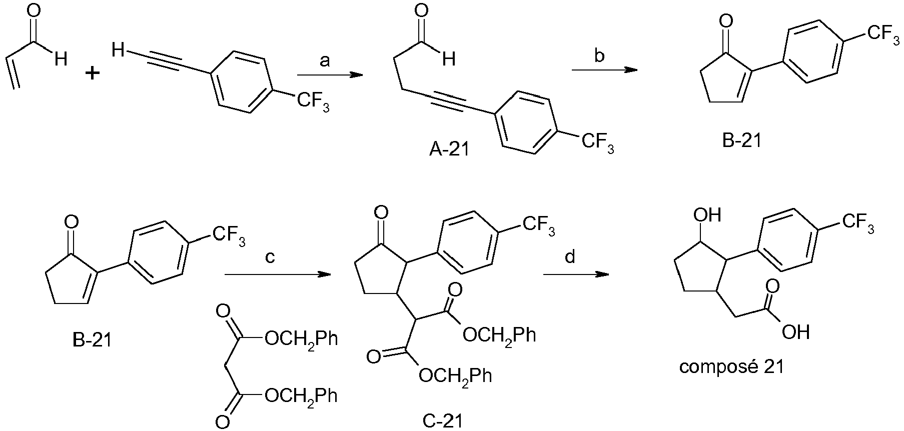

### Etape a :

L'intermédiaire A-21 est obtenu de façon strictement similaire à l'intermédiaire A-5 en substituant le 1-décyne par le ((4-trifluorométhyl)phényl)acétylène, avec un temps de réaction de 17h. Rendement 38 %.

Le spectre RMN ¹H et le spectre de masse sont en accord avec la structure attendue.

### Etape b :

L'intermédiaire A-21 est cyclisé par réaction d'hydroacylation intramoléculaire catalysée par le rhodium selon les conditions décrites par Tanaka et Fu (J. Am. Chem. Soc. 2001, 123, 11492) pour ce type de réaction.

Le complexe de rhodium [Rh(dppe)]₂(BF₄)₂ (0.1 équiv.) et l'intermédiaire A-21 (1 équiv.) sont mis en solution dans l'acétone, dans un tube de Schlenk, sous atmosphère inerte. Le tube est scellé et le mélange réactionnel est agité à une température de 90 °C pendant 16h. Après retour à température ambiante, de l'acétonitrile est ajouté et les solvants sont évaporés sous pression réduite. Le résidu est purifié par chromatographie colonne sur gel de silice pour isoler l'intermédiaire B-21

### Etape c :

5 équivalents du dérivé malonate CH₂(CO₂CH₂Ph)(CO₂CH₂Ph) sont dissous dans le THF.

Puis le dérivé malonate est traité, à une température comprise entre -78 °C et 30 °C, par 5 équivalents d'une base forte : le diéthanolate de magnésium.

Le milieu réactionnel est agité pendant 30 min puis 1 équivalent de la cyclopentéonone B-21 préalablement dissous dans le solvant de réaction est ajouté goutte-à-goutte.

Le mélange réactionnel est agité pendant 4h puis refroidi à une température comprise entre -30 et 0 °C. L'excès de base est neutralisé par ajout d'une solution aqueuse saturée en NH₄Cl et l'adduit C-21 est extrait de 3 fois à l'aide d'acétate d'éthyle. Les phases organiques sont rassemblées, séchées sur Na₂SO₄ et concentrées sous pression réduite. Le résidu est purifié par chromatographie colonne sur gel de silice pour isoler l'intermédiaire C-21.

### Etape d :

L'intermédiaire C-21, préalablement dissous dans l'éthanol, est agité à une température comprise entre 18 et 100 °C, sous atmosphère de dihydrogène en présence d'un catalyseur palladium sur charbon, pendant 4 h. L'hydrogène est ensuite éliminé par passage d'un flux d'argon et le mélange est chauffé à 150 °C pendant 4 heures. Après retour à température ambiante, le catalyseur d'hydrogénation est filtré sur celite. Le filtrat est concentré sous pression réduite et le résidu est purifié par chromatographie colonne sur gel de silice pour l'isoler le composé 21.

### Exemple 6 : préparation du composé 38

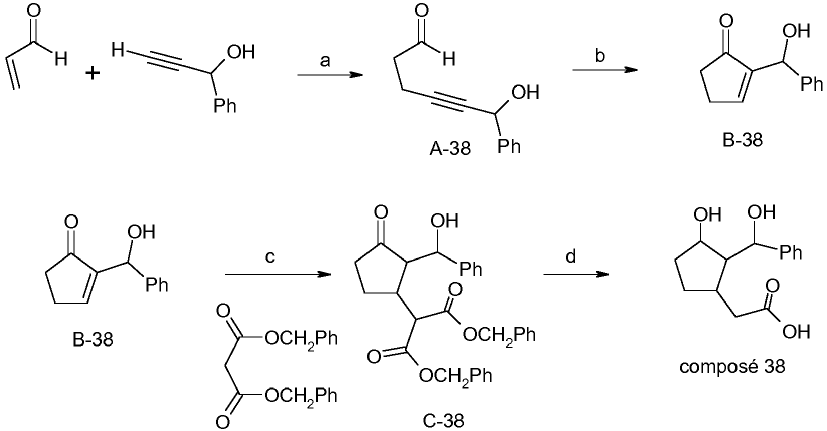

### Etape a :

L'intermédiaire A-38 est obtenu de façon strictement similaire à l'intermédiaire A-5 en substituant le 1-décyne par le 1-hydroxy-1-phényl-prop-2-yne, avec un temps de réaction de 3h. Rendement 43 %.

Le spectre RMN ¹H et le spectre de masse sont en accord avec la structure attendue.

### Etape b :

L'intermédiaire A-38 est cyclisé par réaction d'hydroacylation intramoléculaire catalysée par le rhodium selon les conditions décrites par Tanaka et Fu (J. Am. Chem. Soc. 2001, 123, 11492) pour ce type de réaction.

Le complexe de rhodium [Rh(dppe)]₂(BF₄)₂ (0.1 équiv.) et l'intermédiaire A-38 (1 équiv.) sont mis en solution dans l'acétone, dans un tube de Schlenk, sous atmosphère inerte. Le tube est scellé et le mélange réactionnel est agité à une température de 90 °C pendant 16h. Après retour à température ambiante, de l'acétonitrile est ajouté et les solvants sont évaporés sous pression réduite. Le résidu est purifié par chromatographie colonne sur gel de silice pour isoler l'intermédiaire B-38.

### Etape c :

5 équivalents du dérivé malonate CH₂(CO₂CH₂Ph)(CO₂CH₂Ph) est dissous dans le THF.

Puis le dérivé malonate est traité, à une température comprise entre -78 °C et 30 °C, par 5 équivalents d'une base forte : le diéthanolate de magnésium.

Le milieu réactionnel est agité pendant 30min puis 1 équivalent de la cyclopentéonone B-38 préalablement dissous dans le solvant de réaction sont ajoutés goutte-à-goutte.

Le mélange réactionnel est agité pendant 4h puis refroidi à une température comprise entre -30 et 0 °C. L'excès de base est neutralisé par ajout d'une solution aqueuse saturée en NH₄Cl et l'adduit C-38 est extrait de 3 fois à l'aide d'acétate d'éthyle. Les phases organiques sont rassemblées, séchées sur Na₂SO₄ et concentrées sous pression réduite. Le résidu est purifié par chromatographie colonne sur gel de silice pour isoler l'intermédiaire C-38.

### Etape d :

L'intermédiaire C-38, préalablement dissous dans l'éthanol, est agité à une température comprise entre 18 et 100 °C, sous atmosphère de dihydrogène en présence d'un catalyseur palladium sur charbon, pendant 4 h. L'hydrogène est ensuite éliminé par passage d'un flux d'argon et le mélange est chauffé à 150 °C pendant 4 heures. Après retour à température ambiante, le catalyseur d'hydrogénation est filtré sur celite. Le filtrat est concentré sous pression réduite et le résidu est purifié par chromatographie colonne sur gel de silice pour l'isoler le composé 38.

### Exemple 7 : préparation du composé 1

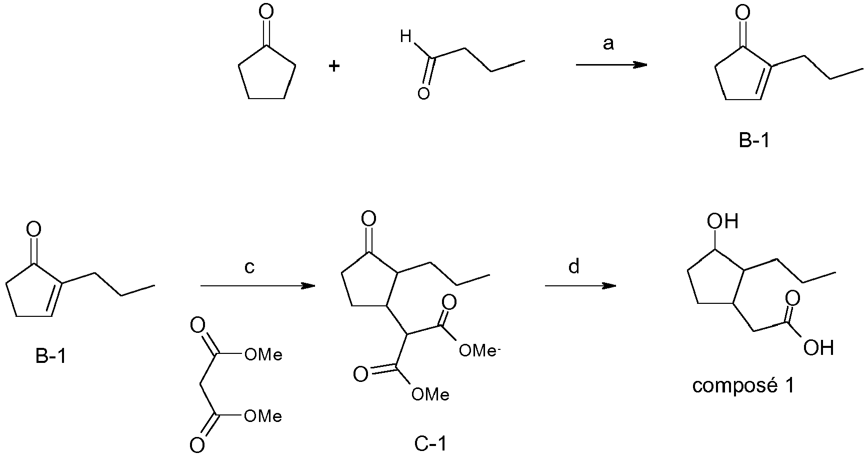

### Etape a :

L'intermédiaire B-1 (CAS : 24105-07-5) est obtenu de façon connue de l'homme de l'art par condensation entre la cyclopentanone et le butyraldéhyde en milieu basique. Rendement 60 %.

Le spectre RMN ¹H et le spectre de masse sont en accord avec la structure attendue.

### Etape c :

5 équivalents du dérivé malonate CH₂(CO₂Me)(CO₂Me) sont dissous dans le méthanol.

Puis le dérivé malonate est traité, à 23 °C, par 5 équivalents d'une base forte : le méthanolate de sodium.

Le milieu réactionnel est agité pendant 6 heures puis 1 équivalent de la cyclopentéonone B-1 préalablement dissoute dans le solvant de réaction est ajoutée goutte-à-goutte.

Le mélange réactionnel est agité pendant 4h. L'excès de base est neutralisé par ajout d'une solution aqueuse saturée en NH₄Cl et l'adduit C-1 est extrait de 3 fois à l'aide d'acétate d'éthyle. Les phases organiques sont rassemblées, séchées sur Na₂SO₄ et concentrées sous pression réduite. Le résidu est purifié par chromatographie colonne sur gel de silice pour isoler l'intermédiaire C-1 (rendement 95%).

### Etape d :

L'intermédiaire C-1, préalablement dissous dans le méthanol, est agité à 23 °C en présence de borohydrure de sodium, ou sous atmosphère de dihydrogène en présence d'un catalyseur palladium sur charbon, pendant 4 h. Une fois cette partie de la réaction terminée et le brut réactionnel traité, le mélange est chauffé à 150 °C pendant 4 heures dans le DMSO. Le filtrat est concentré sous pression réduite et le résidu est purifié par chromatographie colonne sur gel de silice pour l'isoler le composé 1 (40% de rendement).

Le spectre RMN ¹H et le spectre de masse sont en accord avec la structure attendue.

### II. Renouvellement épidermique et effet barrière , ce mode de réalisation n'est pas couvert par les revendications.

L'influence du composé 5, en particulier obtenu selon le procédé de préparation décrit à l'exemple 1 ci-dessus, sur le renouvellement épidermique et l'effet barrière de la peau a été évaluée *in vitro* en mesurant l'expression des transcrits TGM1 et TGM3 dans des kératinocytes.

La transglutaminase est une aminoacyltransférase. Elle est sous forme de polymères de protéines généralement insolubles dans l'eau. Ces polymères biologiques sont indispensables à l'organisme pour créer des barrières et structures stables. Ainsi la transglutaminase est impliquée entre autres dans la synthèse de peau et de cheveux. En particulier la Transglutaminase 3 (TGM3) est une Transglutaminase épidermique (cf Griffin et al. Biochem. J. 2002, vol.368, 377-396).

### Protocole

Les kératinocytes épidermiques humains ont été incubés pendant 24 heures en présence ou non (Témoin) du composé à l'essai. A la fin de l'incubation, les ARNs totaux ont été extraits puis quantifiés. L'expression des transcrits TGM1 et TGM3 a ensuite été mesurée par une methode de RT-qPCR en 2 étapes à l'aide d'un système LightCycler^{®} 480 et selon la technique d'incorporation du SYBR^{®}Green (Qiagen). L'expression de ces transcrits a été normalisée par rapport à l'expression de 2 gènes de ménage, RPL13A et GAPDH. L'expérience a été reproduite 3 fois (N=3).

**Tableau 2 : caractéristiques des amorces utilisées pour l'étape de PCR quantitative :**

| Gène | Abréviation | Gène ID | Nom Qiagen | Réf QIAGEN |
|---|---|---|---|---|
| Transglutaminase 1 | TGM1 | 7051 | Hs_TGM11_SG QuantiTect Primer Assay | QT00082320 |
| Transglutaminase 3 | TGM3 | 7053 | Hs_TGM3_1_SG QuantiTect Primer Assay | QT00001295 |
| Glyceraldehyde 3-phosphate dehydrogenase | GAPDH | 2597 | HS_GAPDH _2_SG QuantiTect Primer Assay | QT01192646 |
| 60S ribosomal protein L13A | RPL13A | 23521 | Hs_RPL13A_2_SG QuantiTect Primer Assay | QT02321333 |

### Résultats

Les résultats sont exprimés en facteur de modulation (fc, fold change) par rapport au témoin.
Stimulation modérée : 1,5 < fc < 2
Stimulation nette : 2 < fc < 3
Stimulation forte : fc > 3
Inhibition modérée : 0,5 < fc < 0,7
Inhibition nette : fc < 0,5

| | **composé 5 (100 µm)** |
|---|---|
| **TGM1** | 1.9 |
| **TGM3** | 1.8 |

Le composé 5 (à 100 µm) a stimulé de façon modérée l'expression des transcrits TGM1 et TGM3.

Ces résultats montrent que le composé 5 selon l'invention présente un effet significatif sur l'augmentation de l'expression des transglutaminases TGM1 et TGM3.

Cette augmentation dans l'expression des transglutaminases traduit un renforcement de la couche cornée de l'épiderme ainsi qu'une amélioration de la fonction barrière permettant de retarder le flétrissement et l'amincissement de la peau.

Ces résultats montrent un effet anti-âge du composé 5 sur la peau notamment par le renforcement de la fonction barrière.

## Revendications

1. Procédé de préparation d'un composé de formule (I) : 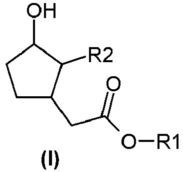 dans laquelle R1 représente un atome d'hydrogène, un radical phényle ou un radical hydrocarboné, linéaire ou ramifié, saturé ou insaturé ayant 1 à 8 atomes de carbone,
R2 désigne un radical choisi parmi :
- un radical hydrocarboné saturé, linéaire, ramifié ou cyclique, ayant 1 à 12 atomes de carbone, éventuellement substitué par 1 à 3 groupements, identiques ou différents, choisis parmi -OR31, -NR31R41, -COOR31, -OCOR31 et les halogènes, avec R31 et R41 représentant, indépendamment l'un de l'autre, un atome d'hydrogène, un radical phényle ou un radical alkyle, linéaire ou ramifié, saturé ou insaturé ayant 1 à 4 atomes de carbone ;
- un radical -Ar-R22 avec Ar est un noyau aromatique choisi parmi les groupes phényle ou pyridyle, et R22 représente un substituant choisi parmi : l'hydrogène, - OR', -NO₂, les halogènes, -NH₂, -CF₃ et -R', avec R' désignant un atome d'hydrogène, un radical hydrocarboné linéaire, ramifié ou cyclique, saturé ou insaturé, ayant 1 à 6 atomes de carbone, ou un radical phényle ;
- un radical -R23-Ph dans lequel R23 représente un radical divalent hydrocarboné linéaire ou ramifié, saturé ou insaturé ayant 1 à 6 atomes de carbone, éventuellement substitué par 1 à 3 groupements -OH et/ou éventuellement interrompu par 1 à 3 atomes d'oxygène non adjacents;
- un radical -R24-NH-R34 avec R24 représente un radical divalent hydrocarboné linéaire ou ramifié, saturé ou insaturé ayant 1 à 4 atomes de carbone, éventuellement substitué par 1 à 3 groupements -OH et/ou éventuellement interrompu par 1 à 3 atomes d'oxygène ; R34 représente un substituant -COOR' ou un radical hydrocarboné, linéaire ou ramifié, saturé ou insaturé ayant 1 à 4 atomes de carbone éventuellement interrompu par un groupement NH, O, S, avec R' représente un atome d'hydrogène, un radical hydrocarboné saturé linéaire, ramifié ou cyclique, ayant 1 à 6 atomes de carbone, ou un radical phényle
- un radical -CO-O-R25 dans lequel R25 représente un atome d'hydrogène, un radical phényle ou un radical hydrocarboné, linéaire ou ramifié, saturé ou insaturé ayant 1 à 8 atomes de carbone ;
ainsi que leurs isomères optiques, diastéréoisomères et/ou sels correspondants ;
procédé dans lequel les étapes suivantes sont mises en œuvre :
a) addition 1,4 sur l'acroléine d'un alcyne de formule R2C=CH avec R2 tel que défini dans la formule (I) ci-dessus;
b) cyclisation du produit obtenu à l'issu de l'étape a) par hydroacylation intramoléculaire ;
c) addition 1,4 sur la cyclopenténone issue de l'étape b) du dérivé malonate CH₂(CO₂CH₂Ph)(CO₂R0) pour lequel, lorsque dans la formule (I) R1 est un atome d'hydrogène, alors -R0 est -CH₂Ph et lorsque dans la formule (I) R1n'est pas un atome d'hydrogène alors R0 est R1 ;
d) réduction et décarboxylation du produit obtenu à l'étape c).

2. Procédé selon la revendication 1, dans lequel le composé est de formule (I) dans laquelle :
R1 est tel que défini à la revendication 1, de préférence R1 est l'hydrogène ou un groupe éthyle, et
R2 désigne un radical hydrocarboné saturé linéaire ayant 3 à 12, de carbone substitué par 1 à 3 groupements, identiques ou différents, choisis parmi -OR31, - NHR31, -COOR31,-OCOR31 et les halogènes avec R31 représentant un atome d'hydrogène, un radical phényle ou un radical alkyle, linéaire ou ramifié, saturé ou insaturé ayant 1 à 4 atomes de carbone, ou
R2 désigne un radical choisi parmi un radical hydrocarboné saturé cyclique ayant 5 à 7 atomes de carbone pouvant être substitué par 1 à 3 groupements, identiques ou différents, choisis parmi -OR31, -NHR31, -COOR31,-OCOR31 et les halogènes avec R31 représentant un atome d'hydrogène, un radical phényle ou un radical alkyle, linéaire ou ramifié, saturé ou insaturé ayant 1 à 4 atomes de carbone ;
R2 désigne un radical hydrocarboné saturé ramifié ayant 3 à 12 atomes de carbone éventuellement substitué par 1 à 3 groupements, identiques ou différents, choisis parmi -OR31, -NHR31, -COOR31,-OCOR31 et les halogènes avec R31 représentant un atome d'hydrogène, un radical phényle ou un radical alkyle, linéaire ou ramifié, saturé ou insaturé ayant 1 à 4 atomes de carbone.

3. Procédé selon l'une quelconque des revendications précédentes, dans lequel le composé de formule (I) est choisi parmi les composés suivants :

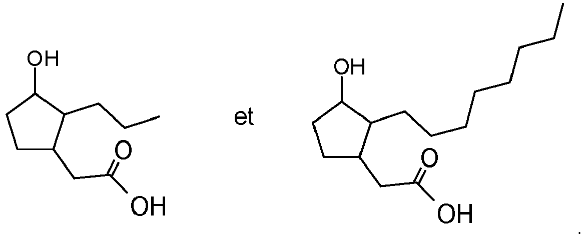

4. Procédé selon l'une quelconque des revendications précédentes, dans lequel le composé est de formule (I) dans laquelle :
R1 est tel que défini à la revendication 1 de préférence R1 est l'hydrogène ou un groupe éthyle, et R2 désigne est un radical -Ar-R22 avec Ar est un noyau aromatique phényle ou pyridyle et
R22 représente un substituant du noyau aromatique Ar choisi parmi l'hydrogène, - OR', -NO₂, -F, -Br, -NH2, -CF3, -R', avec R' représente un atome d'hydrogène, un radical hydrocarboné saturé linéaire, ramifié ou cyclique, ayant 1 à 4 atomes de carbone ou un radical phényle.

5. Procédé selon l'une quelconque des revendications précédentes, dans lequel le composé est de formule (I) dans laquelle :
R1 est tel que défini à la revendication 1, de préférence R1 est l'hydrogène ou un groupe éthyle, et R2 est un radical -R23Ph avec R23 représente un radical hydrocarboné linéaire saturé ou insaturé ayant 1 à 6 atomes de carbone, éventuellement substitué par 1 groupement -OH et/ou éventuellement interrompu par 1 atome d'oxygène.

6. Procédé selon l'une quelconque des revendications précédentes, dans lequel le composé est de formule (I) dans laquelle :
R1 est tel que défini à la revendication 1, de préférence R1 est l'hydrogène ou un groupe éthyle, et R2 est un radical -R24-NH-R34 avec R24 représente un radical hydrocarboné divalent, linéaire, saturé ayant 1 à 4 atomes de carbone et R34 représente un substituant -COOR' ou un radical hydrocarboné, linéaire saturé ayant 1 à 4 atomes de carbone éventuellement interrompu par un atome d'oxygène, R' représente un radical hydrocarboné saturé linéaire, ramifié, ayant 1 à 4 atomes de carbone.

7. Procédé selon l'une quelconque des revendications précédentes, dans lequel le composé est de formule (I) dans laquelle :
R1 est tel que défini à la revendication 1, de préférence R1 est l'hydrogène ou un groupe éthyle, et R2 est un radical -CO-O-R25 et -R25 représente, un atome d'hydrogène ou un radical hydrocarboné saturé, linéaire ou ramifié ayant 1 à 4 atomes de carbone.

8. Procédé selon la revendication 1 dans lequel l'étape a) est effectuée en présence d'un catalyseur qui est un complexe de palladium en particulier choisi parmi Pd(OAc)2 , Pd(TFA)2, Pd(OPiv)2, PdCl2, Pd2dba3 éventuellement associé à une phosphine ou complexe de rhodium éventuellement associé à une phosphine.

9. Procédé selon la revendication 8 dans lequel ledit catalyseur est Pd(OAc)2 et la phosphine est PMe3, et en particulier mis en présence dans un rapport molaire Pd(OAc)2 / PMe3 d'environ 1/3.

10. Procédé selon l'une quelconque des revendications précédentes dans lequel à l'étape a), l'alcyne et l'acroléine sont chauffés à une température comprise entre 30 °C et 140 °C dans un solvant organique qui est de préférence un mélange d'au moins 2 solvants miscibles ou non choisis parmi le toluène, le benzène, l'eau, le méthanol, l'éthanol, l'isopropanol, l'acétone, la méthyléthylcétone, le THF, le Me-THF, l'éther diéthylique, l'éther diisopropylique, le dichlorométhane et l'acétonitrile.

## Patentansprüche

1. Verfahren zur Herstellung einer Verbindung von Formel (I): 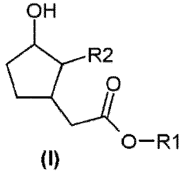 wobei R1 ein Wasserstoffatom, ein lineares oder verzweigtes, gesättigtes oder ungesättigtes Phenyl-Radikal oder Kohlenwasserstoff-Radikal darstellt, das 1 bis 8 Kohlenstoffatome aufweist,
R2 ein Radikal bezeichnet, ausgewählt aus:
- ein gesättigtes, lineares, verzweigtes oder cyclisches Kohlenwasserstoff-Radikal, das 1 bis 12 Kohlenstoffatome aufweist, optional substituiert mit 1 bis 3 gleichen oder verschiedenen Gruppierungen, ausgewählt aus -OR31, -NR31R41, -COOR31, - OCOR31 und Halogenen, wobei R31 und R41 unabhängig voneinander ein Wasserstoffatom, ein Phenyl-Radikal oder ein lineares oder verzweigtes, gesättigtes oder ungesättigtes Alkyl-Radikal darstellen, das 1 bis 4 Kohlenstoffatome aufweist;
- ein Radikal -Ar-R22, wobei Ar ein aromatischer Ring ist, ausgewählt aus Phenyl oder Pyridyl, und R22 einen Substituenten darstellt, ausgewählt aus: Wasserstoff, -OR', -NO₂, Halogenen, -NH₂, -CF₃ und -R', wobei R' ein Wasserstoffatom, ein lineares, verzweigtes oder cyclisches, gesättigtes oder ungesättigtes Kohlenwasserstoff-Radikal, das 1 bis 6 Kohlenstoffatome aufweist oder ein Phenyl-Radikal bezeichnet;
- ein Radikal -R23-Ph, wobei R23 einen zweiwertiges lineares oder verzweigtes, gesättigtes oder ungesättigtes Kohlenwasserstoff-Radikal darstellt, das 1 bis 6 Kohlenstoffatome aufweist, optional substituiert mit 1 bis 3 -OH-Gruppierungen und/oder optional substituiert mit 1 bis 3 nicht angrenzenden Sauerstoffatomen;
- ein Radikal -R24-NH-R34, wobei R24 ein zweiwertiges, lineares oder verzweigtes, gesättigtes oder ungesättigtes Kohlenwasserstoff-Radikal darstellt, das 1 bis 4 Kohlenstoffatome aufweist, optional substituiert mit 1 bis 3 Gruppierungen -OH und/oder optional unterbrochen durch 1 bis 3 Sauerstoffatome; R34 einen Substituenten -COOR' oder ein lineares oder verzweigtes, gesättigtes oder ungesättigtes Kohlenwasserstoff-Radikal darstellt, das 1 bis 4 Kohlenstoffatome aufweist, optional unterbrochen durch eine Gruppe NH, O, S, wobei R' ein Wasserstoffatom, einen lineares, verzweigtes oder cyclischen, gesättigtes Kohlenwasserstoff-Radikal, das 1 bis 6 Kohlenstoffatome aufweist, oder ein Phenyl-Radikal darstellt
- ein Radikal -CO-O-R25, wobei R25 ein Wasserstoffatom, ein Phenyl-Radikal oder ein lineares oder verzweigtes, gesättigtes oder ungesättigtes Kohlenwasserstoff-Radikal darstellt, das 1 bis 8 Kohlenstoffatomen aufweist;
sowie ihre entsprechenden optischen Isomere, Diastereomere und/oder Salze; Verfahren, bei dem die folgenden Schritte durchgeführt werden:
a) 1,4-Addition eines Alkins von Formel R2C=CH an Acrolein, wobei R2 wie definiert in der obigen Formel (I) definiert;
b) Cyclisieren des von Schritt a) erlangten Produkts durch intramolekulare Hydroacylierung;
c) 1,4-Addition an Cyclopentenon von Schritt b) des Malonatderivats CH₂(CO₂CH₂Ph)(CO₂R0), wobei, wenn in Formel (I) R1 ein Wasserstoffatom ist, dann - R0 -CH₂Ph ist, und wenn in Formel (I) R1 kein Wasserstoffatom ist, dann R0 R1 ist;
d) Reduzieren und Decarboxylieren in Schritt c) erlangten Produkts.

2. Verfahren nach Anspruch 1, wobei die Verbindung aus Formel (I) besteht, wobei:
R1 wie definiert in Anspruch 1 ist, vorzugsweise R1 Wasserstoff oder eine Ethylgruppe ist, und
R2 ein lineares gesättigtes Kohlenwasserstoff-Radikal bezeichnet, das 3 bis 12 Kohlenstoffatome aufweist, substituiert mit 1 bis 3 gleichen oder verschiedenen Gruppierungen, ausgewählt aus -OR31, -NHR31, -COOR31, -OCOR31 und Halogenen, wobei R31 ein Wasserstoffatom, ein lineares oder verzweigtes, gesättigtes oder ungesättigtes Phenyl-Radikal oder ein Alkyl-Radikal darstellt, das 1 bis 4 Kohlenstoffatome aufweist, oder
R2 ein Radikal bezeichnet, ausgewählt aus einem cyclischen gesättigten Kohlenwasserstoff-Radikal, das 5 bis 7 Kohlenstoffatome aufweist, das mit 1 bis 3 gleichen oder verschiedenen Gruppen substituiert sein kann, ausgewählt aus -OR31, - NHR31, -COOR31,-OCOR31 und Halogenen, wobei R31 ein Wasserstoffatom, ein lineares oder verzweigtes, gesättigtes oder ungesättigtes Phenyl-Radikal oder ein Alkyl-Radikal darstellt, das 1 bis 4 Kohlenstoffatome aufweist;
R2 ein verzweigtes gesättigtes Kohlenwasserstoff-Radikal mit 3 bis 12 Kohlenstoffatomen bezeichnet, optional substituiert mit 1 bis 3 gleichen oder verschiedenen Gruppierungen, ausgewählt aus -OR31, -NHR31, -COOR31,-OCOR31 und Halogenen, wobei R31 ein Wasserstoffatom, ein lineares oder verzweigtes, gesättigtes oder ungesättigtes Phenyl-Radikal oder ein Alkyl-Radikal darstellt, das 1 bis 4 Kohlenstoffatome aufweist.

3. Verfahren nach einem der vorherigen Ansprüche, wobei die Verbindung von Formel (I) ausgewählt ist aus den folgenden Verbindungen:

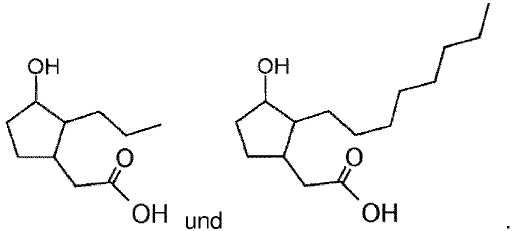

4. Verfahren nach einem der vorherigen Ansprüche, wobei die Verbindung von Formel (I) ist, wobei:
R1 wie definiert in Anspruch 1 ist, vorzugsweise R1 Wasserstoff oder eine Ethylgruppe ist, und R2 ein Radikal -Ar-R22 bezeichnet, wobei Ar ein aromatischer Phenyl- oder Pyridylring ist, und
R22 einen Substituenten des aromatischen Rings Ar darstellt, ausgewählt aus Wasserstoff, -OR', -NO₂, -F, -Br, -NH2, -CF3, -R', wobei R' ein Wasserstoffatom, einen lineares, verzweigtes oder cyclisches gesättigtes Kohlenwasserstoff-Radikal, das 1 bis 4 Kohlenstoffatome aufweist, oder ein Phenyl-Radikal darstellt.

5. Verfahren nach einem der vorherigen Ansprüche, wobei die Verbindung von Formel (I) ist, wobei:
R1 wie definiert in Anspruch 1 ist, vorzugsweise R1 Wasserstoff oder eine Ethylgruppe ist, und R2 ein Radikal -R23Ph ist, wobei R23 ein gesättigtes oder ungesättigtes lineares Kohlenwasserstoff-Radikal darstellt, das 1 bis 6 Kohlenstoffatome aufweist, optional substituiert mit 1 -OH-Gruppierung und/oder optional unterbrochen durch 1 Sauerstoffatom.

6. Verfahren nach einem der vorherigen Ansprüche, wobei die Verbindung von Formel (I) ist, wobei:
R1 wie definiert in Anspruch 1 ist, vorzugsweise R1 Wasserstoff oder eine Ethylgruppe ist, und R2 ein Radikal -R24-NH-R34 ist, wobei R24 ein zweiwertiges, lineares, gesättigtes Kohlenwasserstoff-Radikal darstellt, das 1 bis 4 Kohlenstoffatome aufweist, und R34 einen Substituenten -COOR' oder einen lineares, gesättigtes Kohlenwasserstoff-Radikal darstellt, das 1 bis 4 Kohlenstoffatome aufweist, der optional unterbrochen durch ein Sauerstoffatom, R' einen lineares, verzweigtes, gesättigtes Kohlenwasserstoff-Radikal darstellt, das 1 bis 4 Kohlenstoffatome aufweist.

7. Verfahren nach einem der vorherigen Ansprüche, wobei die Verbindung von Formel (I) ist, wobei:
R1 ist wie in Anspruch 1 definiert, vorzugsweise ist R1 Wasserstoff oder eine Ethylgruppe und R2 ist ein Radikal -CO-O-R25 und -R25 ist ein Wasserstoffatom oder ein gesättigter, geradkettiger oder verzweigter Kohlenwasserstoff-Radikal, das 1 bis 4 Kohlenstoffatome aufweist.

8. Verfahren nach Anspruch 1, wobei Schritt a) in Anwesenheit eines Katalysators ausgeführt wird, der ein Palladiumkomplex ist, insbesondere ausgewählt aus Pd(OAc)2 Pd(TFA)2, Pd(OPiv)2, PdCl2, Pd2dba3, optional assoziiert mit einem Phosphin, oder einem Rhodiumkomplex, optional assoziiert mit einem Phosphin.

9. Verfahren nach Anspruch 8, wobei der Katalysator Pd(OAc)2 ist und das Phosphin PMe3 ist, und insbesondere in einem Molverhältnis Pd(OAc)2/PMe3 von etwa 1/3 zusammengebracht wird.

10. Verfahren nach einem der vorherigen Ansprüche, wobei in Schritt a) Alkin und das Acrolein auf eine Temperatur zwischen 30 °C und 140 °C in einem organischen Lösungsmittel erhitzt werden, das vorzugsweise ein Gemisch aus mindestens 2 mischbaren oder nicht mischbaren Lösungsmitteln ist, ausgewählt aus Toluol, Benzol, Wasser, Methanol, Ethanol, Isopropanol, Aceton, Methylethylketon, THF, Me-THF, Diethylether, Diisopropylether, Dichlormethan und Acetonitril.

## Claims

1. A method for preparing a compound of formula (I): 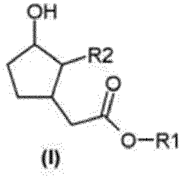 wherein R1 represents a hydrogen atom, a phenyl radical or a linear or branched, saturated or unsaturated hydrocarbon-based radical containing 1 to 8 carbon atoms,
R2 denotes a radical chosen from:
- a linear, branched or cyclic, saturated hydrocarbon-based radical containing 1 to 12 carbon atoms, optionally substituted with 1 to 3 groups, which may be identical or different, chosen from -OR31, -NR31R41, -COOR31, -OCOR31 and halogens, with R31 and R41 representing, independently of one another, a hydrogen atom, a phenyl radical or a linear or branched, saturated or unsaturated alkyl radical containing 1 to 4 carbon atoms;
- a radical -Ar-R22, with Ar being an aromatic ring chosen from phenyl or pyridyl groups, and R22 representing a substituent chosen from: hydrogen, -OR', -NO₂, halogens, -NH₂, -CF₃ and -R', with R' denoting a hydrogen atom, a linear, branched or cyclic, saturated or unsaturated hydrocarbon-based radical containing 1 to 6 carbon atoms, or a phenyl radical;
- a radical -R23-Ph, wherein R23 represents a linear or branched, saturated or unsaturated hydrocarbon-based divalent radical containing 1 to 6 carbon atoms, optionally substituted with 1 to 3 -OH groups and/or optionally interrupted with 1 to 3 non-adjacent oxygen atoms;
- a radical -R24-NH-R34, wherein R24 represents a linear or branched, saturated or unsaturated hydrocarbon-based divalent radical containing 1 to 4 carbon atoms, optionally substituted with 1 to 3 -OH groups and/or optionally interrupted with 1 to 3 oxygen atoms; R34 represents a substituent -COOR' or a linear or branched, saturated or unsaturated hydrocarbon-based radical containing 1 to 4 carbon atoms, optionally interrupted with an NH, O or S group, wherein R' represents a hydrogen atom, a linear, branched or cyclic, saturated hydrocarbon-based radical containing 1 to 6 carbon atoms, or a phenyl radical;
- a radical -CO-O-R25, wherein R25 represents a hydrogen atom, a phenyl radical or a linear or branched, saturated or unsaturated hydrocarbon-based radical containing 1 to 8 carbon atoms;
as well as the optical isomers, diastereomers and/or corresponding salts thereof;
in which method the following steps are carried out:
a) 1,4 addition to acrolein of an alkyne of formula R2C=CH with R2 as defined in formula (I) above;
b) cyclization of the product obtained at the end of step a) by intramolecular hydroacylation;
c) 1,4 addition to the cyclopentenone resulting from step b) of the malonate derivative CH₂ (CO₂CH₂Ph) (CO₂R0) for which, when in formula (I) R1 is a hydrogen atom, then -R0 is -CH₂Ph and when in formula (I) R1 is not a hydrogen atom, then R0 is R1;
d) reduction and decarboxylation of the product obtained in step c).

2. The method as claimed in claim 1, wherein the compound is of formula (I), wherein:
R1 is as defined in claim 1, preferably R1 is hydrogen or an ethyl group, and
R2 denotes a linear, saturated hydrocarbon-based radical containing 3 to 12 carbon atoms, substituted with 1 to 3 groups, which may be identical or different, chosen from -OR31, -NHR31, -COOR31, -OCOR31 and halogens, with R31 representing a hydrogen atom, a phenyl radical or a linear or branched, saturated or unsaturated alkyl radical containing 1 to 4 carbon atoms, or
R2 denotes a radical chosen from a cyclic, saturated hydrocarbon-based radical containing 5 to 7 carbon atoms, possibly substituted with 1 to 3 groups, which may be identical or different, chosen from -OR31, -NHR31, - COOR31, -OCOR31 and halogens, with R31 representing a hydrogen atom, a phenyl radical or a linear or branched, saturated or unsaturated alkyl radical containing 1 to 4 carbon atoms;
R2 denotes a branched, saturated hydrocarbon-based radical containing 3 to 12 carbon atoms, optionally substituted with 1 to 3 groups, which may be identical or different, chosen from -OR31, -NHR31, -COOR31, -OCOR31 and halogens, with R31 representing a hydrogen atom, a phenyl radical or a linear or branched, saturated or unsaturated alkyl radical containing 1 to 4 carbon atoms.

3. The method as claimed in either of the preceding claims, wherein the compound of formula (I) is chosen from the following compounds:

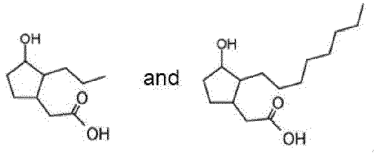

4. The method as claimed in any one of the preceding claims, wherein the compound is of formula (I), wherein:
R1 is as defined in claim 1, preferably R1 is hydrogen or an ethyl group, and R2 denotes a radical -Ar-R22 with Ar being a phenyl or pyridyl aromatic nucleus and
R22 represents a substituent of the aromatic nucleus Ar chosen from hydrogen, -OR', -NO₂, -F, -Br, -NH₂, -CF₃ and -R', with R' representing a hydrogen atom, a linear, branched or cyclic, saturated hydrocarbon-based radical containing 1 to 4 carbon atoms or a phenyl radical.

5. The method as claimed in any one of the preceding claims, wherein the compound is of formula (I), wherein R1 is as defined in claim 1, preferably R1 is hydrogen or an ethyl group, and R2 is a radical -R23Ph with R23 representing a linear saturated or unsaturated hydrocarbon-based radical containing 1 to 6 carbon atoms, optionally substituted with 1 -OH group and/or optionally interrupted with 1 oxygen atom.

6. The method as claimed in any one of the preceding claims, wherein the compound is of formula (I), wherein:
R1 is as defined in claim 1, preferably R1 is hydrogen or an ethyl group, and R2 is a radical -R24-NH-R34 with R24 representing a linear, saturated hydrocarbon-based divalent radical containing 1 to 4 carbon atoms and R34 representing a substituent -COOR' or a linear saturated hydrocarbon-based radical containing 1 to 4 carbon atoms, optionally interrupted with an oxygen atom, R' representing a linear or branched, saturated hydrocarbon-based radical containing 1 to 4 carbon atoms.

7. The method as claimed in any one of the preceding claims, wherein the compound is of formula (I), wherein:
R1 is as defined in claim 1, preferably R1 is hydrogen or an ethyl group, and R2 is a radical -CO-O-R25 and -R25 represents a hydrogen atom or a linear or branched, saturated hydrocarbon-based radical containing 1 to 4 carbon atoms.

8. The method as claimed in claim 1, wherein step a) is carried out in the presence of a catalyst which is a palladium complex in particular chosen from Pd(OAc)₂, Pd(TFA)₂, Pd(OPiv)₂, PdCl₂ and Pd₂dba₃ optionally combined with a phosphine or a rhodium complex optionally combined with a phosphine.

9. The method as claimed in claim 8, wherein said catalyst is Pd(OAc)₂ and the phosphine is PMe₃, and in particular brought into contact in a Pd(OAc)₂ / PMe₃ mole ratio of approximately 1/3.

10. The method as claimed in any one of the preceding claims, wherein, in step a), the alkyne and the acrolein are heated to a temperature of between 30°C and 140°C in an organic solvent which is preferably a mixture of at least 2 miscible or immiscible solvents chosen from toluene, benzene, water, methanol, ethanol, isopropanol, acetone, methyl ethyl ketone, THF, Me-THF, diethyl ether, diisopropyl ether, dichloromethane and acetonitrile.
